(19) Europäisches Patentamt
European Patent Office
Office européen des brevets

(11) EP 4 338 702 A1

(12) EUROPÄISCHE PATENTANMELDUNG

(43) Veröffentlichungstag:
**20.03.2024 Patentblatt 2024/12**

(21) Anmeldenummer: **23195901.6**

(22) Anmeldetag: **07.09.2023**

(51) Internationale Patentklassifikation (IPC):
***A61C 7/08*** *(2006.01)* ***A61K 6/62*** *(2020.01)*
***A61K 6/887*** *(2020.01)* ***B33Y 70/00*** *(2020.01)*
***B33Y 80/00*** *(2015.01)*

(52) Gemeinsame Patentklassifikation (CPC):
(C-Sets verfügbar)
**A61K 6/887; A61C 7/08; A61K 6/62; B33Y 70/00; B33Y 80/00** (Forts.)

(84) Benannte Vertragsstaaten:
**AL AT BE BG CH CY CZ DE DK EE ES FI FR GB GR HR HU IE IS IT LI LT LU LV MC ME MK MT NL NO PL PT RO RS SE SI SK SM TR**
Benannte Erstreckungsstaaten:
**BA**
Benannte Validierungsstaaten:
**KH MA MD TN**

(30) Priorität: **15.09.2022 DE 102022123585**

(71) Anmelder: **VOCO GmbH**
**27472 Cuxhaven (DE)**

(72) Erfinder:
- **Lamping, Sebastian**
  **48153 Münster (DE)**
- **Nordmann, Markus**
  **27472 Cuxhaven (DE)**
- **Oldenburger, Daniel**
  **27476 Cuxhaven (DE)**
- **Plaumann, Manfred Thomas**
  **27472 Cuxhaven (DE)**

# (54) UNREAKTIVE TRANSFERSCHIENEN

(57) Die vorliegende Erfindung betrifft radikalisch polymerisierbare Zusammensetzungen sowie deren Verwendung in Stereolithographie und/oder 3D-Druck, insbesondere zum 3D-Druck von dentalen Formteilen wie dentalen Transferschienen, IBTs, Übertragungsschlüsseln und Bohrschablonen. Die radikalisch polymerisierbaren Zusammensetzungen zeichnen sich dadurch aus, dass sie nach der Polymerisation praktisch keine Haftung zu anderen (Meth)acryl-Systemen aufweisen.

EP 4 338 702 A1

(52) Gemeinsame Patentklassifikation (CPC): (Forts.)

C-Sets
**A61K 6/887, C08L 33/08;**
**A61K 6/887, C08L 33/10**

## Beschreibung

Die vorliegende Erfindung betrifft eine radikalisch polymerisierbare Zusammensetzung sowie deren Verwendung in Stereolithographie und/oder 3D-Druck, vorzugsweise zum 3D-Druck von dentalen Formteilen wie dentalen Transferschienen, IBTs, Übertragungsschlüsseln und Bohrschablonen.

Die Erfindung wird in den beigefügten Patentansprüchen definiert. Bevorzugte Aspekte der vorliegenden Erfindung ergeben sich überdies aus der nachfolgenden Beschreibung einschließlich der Beispiele.

Soweit für einen erfindungsgemäßen Aspekt (Zusammensetzung oder Verwendung) bestimmte Ausgestaltungen als bevorzugt bezeichnet werden, gelten die entsprechenden Ausführungen jeweils auch für die anderen Aspekte der vorliegenden Erfindung, *mutatis mutandis*. Bevorzugte individuelle Merkmale erfindungsgemäßer Aspekte (wie in den Ansprüchen definiert und/oder in der Beschreibung offenbart) sind miteinander kombinierbar und werden vorzugsweise miteinander kombiniert, sofern sich im Einzelfall für den Fachmann aus dem vorliegenden Text nichts anderes ergibt.

Mit der stetigen Weiterentwicklung der digitalen Zahnmedizin haben sich generative Fertigungsverfahren inzwischen sowohl in Zahnarztpraxen als auch in Dentallabors etabliert. Daten der individuellen Zahnsituation werden oftmals durch 3D-Scanner gewonnen und können am Computer bearbeitet werden. Mittels 3D-Druck oder Stereolithographie können auf Basis dieser Daten dann je nach Anwendung dentale Formteile wie beispielsweise Modelle, Trays, Dentures, Splints, Schienen, orthodontische Formteile, Indirect-Bonding-Trays (IBTs), Casts, Bohrschablonen, Kronen oder Brücken gedruckt werden.

Generative Fertigungsverfahren, speziell auch stereolithographische Verfahren zur Herstellung dentaler Formkörper, sind aus dem Stand der Technik bekannt.

In der WO 97/29901 A1 wird ein Verfahren zur Herstellung eines dreidimensionalen Gegenstandes aus einem härtbaren flüssigen Medium angegeben, wobei der Gegenstand Schicht für Schicht aufgebaut wird, indem jedes Mal eine Schicht flüssigen Mediums auf einen Träger und/oder einen bereits geformten Teil des Gegenstandes in einem flüssiges Medium enthaltenden Behälter angebracht wird und anschließend besagte Schicht gehärtet wird.

Die WO 2013/153183 A2 beschreibt Kompositharzzusammensetzungen und Verfahren zur Herstellung dentaler Bauteile mittels Stereolithographie. Beansprucht wird die Verwendung einer Dentalzusammensetzung, umfassend ein polyreaktives Bindemittel, zwei Photopolymerisationsinitiatoren mit unterschiedlichen Absorptionsmaxima und einen Absorber.

Polymerisierbare Zusammensetzungen, insbesondere (Meth)acylat-Zusammensetzungen, weisen üblicherweise einen gewissen Anteil an nicht umgesetzten Doppelbindungen auf. Dies hat den Vorteil, dass (Meth)acrylat-Zusammensetzungen sich sehr gut mit bereits polymerisierten (Meth)acrylat-Zusammensetzungen verbinden. Dies wird u.a. in der direkten Füllungstherapie genutzt, wo z.B. ein Füllungsmaterial sehr gut auf einem photopolymerisierten Bonding haftet. Auch die Inkrementtechnik beruht darauf, dass ein Inkrement jeweils auf dem vorherigen, polymerisierten Inkrement optimal haftet und so eine homogene Kompositfüllung ergibt.

Es gibt aber auch Anwendungen, in denen eine Haftung bzw. ein Verbund gerade nicht erwünscht ist. Abdrücke, Übertragungsschlüssel, Transferschienen, IBTs oder Schablonen dürfen keine Bindung zu dem mit ihnen verarbeiten Material eingehen. Sollen daher (Meth)acryl-Systeme verarbeitet werden, werden häufig Silikone eingesetzt.

Gleichzeitig besteht aber der Wunsch auch Abdrücke, Übertragungsschlüssel, Transferschienen, IBTs oder Schablonen mittels Photopolymerisation durch 3D-Druck auf Basis von präzisen 3D-Daten herzustellen.

Es besteht also der Bedarf an radikalisch polymerisierbaren Zusammensetzungen, insbesondere (Meth)acryl-Zusammensetzungen, die nach der Polymerisation keine oder keine nennenswerte Haftung zu anderen (Meth)acryl-Zusammensetzungen aufweisen.

Gelöst wird die Aufgabe durch eine dentale, radikalisch polymerisierbare Zusammensetzung umfassend

A1) eine oder mehrere polymerisierbare (Meth)acryl-Verbindungen der Formel I

$$Y\text{-}O\text{-}[(C_3H_6O)_v\text{-}L^1]_n\text{-}(C_3H_6O)_w\text{-}Z \qquad (I)$$

wobei

$Y = H_2C{=}C(R^1)\text{-}C({=}O)\text{-}$ oder $H_2C{=}C(R^1)\text{-}C({=}O)\text{-}X\text{-}L^2\text{-}$,
$R^1$ = H oder $CH_3$,
X = O oder NH,
$L^1$ = divalente organische Linkergruppe,
$L^2$ = divalente organische Linkergruppe,
Z = Y, H oder C1 bis C4-Alkyl,
v = 36 bis 100,

w = 36 bis 200,
n = 0 bis 3,

$$n*v+w = 36 \text{ bis } 200$$

A2) eine oder mehrere monofunktionelle (Meth)acryl-Verbindungen mit einer (Meth)acryl-Gruppe, die nicht der Formel I entsprechen,
A3) eine oder mehrere difunktionelle (Meth)acryl-Verbindungen mit zwei (Meth)acryl-Gruppen, die nicht der Formel I entsprechen und
B) einen Photoinitiator oder ein Photoinitiatorsystem.

Unter (Meth)acryl-Verbindungen werden dabei sowohl Acryl-Verbindungen als auch Methacryl-Verbindungen verstanden.
In einer bevorzugten Ausführungsform umfasst eine erfindungsgemäße Zusammensetzung zusätzlich eine oder mehrere multifunktionelle (Meth)acryl-Verbindungen (A4) mit drei bis sechs (Meth)acryl-Gruppen.
An sich ist der Einsatz von Monomeren mit Polypropylengruppen im 3D-Druck bekannt. So beschreibt die WO 2019/175716 A1 mehrere entsprechende Monomere. Allerdings zeigen die dort genannten Monomere nicht den gewünschten Effekt. Wahrscheinlich sind die PPG-Einheiten nicht groß genug.
Auch die WO 2022/097667 A1 beschreibt lichthärtbare Harzzusammensetzungen mit Polyalkylenglycol-Monomeren. Die Zusammensetzungen werden für die Herstellung von Modellen in Gießverfahren eingesetzt und zeichnen sich durch geringe Rußentwicklung aus, was zur Reduzierung von Sprüngen führen soll. Längerkettige Monomere sind nur in Zusammenhang mit PEG-Einheiten offenbart.
Überraschenderweise hat sich gezeigt, dass die Kombination einer (Meth)acryl-Verbindung (A1) der Formel (I), die eine oder mehrere große PPG-Gruppen aufweist, mit den (Meth)acryl-Verbindungen (A2), (A3) und ggf. (A4) sowie einem Photoinitiator (B) zu Zusammensetzungen führt, die nach der Polymerisation praktisch keine Haftung zu anderen (Meth)acryl-Systemen aufweisen.
Offensichtlich ergibt sich dabei ein synergistischer Effekt von den PPG-Domänen auf der einen Seite und von der Kombination aus mono-, di- und multifunktionellen Monomeren auf der anderen Seite. Während die di- und multifunktionellen Monomere ein dreidimensionales Netzwerk um die PPG-Domänen aufbauen, führen die mobilen monofunktionellen Monomere zu einer weitgehend vollständigen Absättigung der noch freien Doppelbindungen des Netzwerks. Auf diese Weise entsteht ein Formkörper, der gegenüber weiteren (Meth)acryl-Monomeren im Wesentlichen unreaktiv ist.

Polymerisierbare (Meth)acryl-Verbindung (A1)

Eine erfindungsgemäße Zusammensetzung enthält 30 bis 70 Gew.-%, bevorzugt 40 bis 60 Gew.-%, polymerisierbare (Meth)acryl-Verbindungen der Formel I

$$Y\text{-}O\text{-}[(C_3H_6O)_v\text{-}L^1]_n\text{-}(C_3H_6O)_w\text{-}Z \qquad (I)$$

wobei

Y = $H_2C=C(R^1)-C(=O)-$ oder $H_2C=C(R^1)-C(=O)-X-L^2-$,
$R^1$ = H oder $CH_3$,
X = O oder NH,
$L^1$ = divalente organische Linkergruppe,
$L^2$ = divalente organische Linkergruppe,
Z = Y, H oder C1 bis C4-Alkyl,
v = 36 bis 100,
w = 36 bis 200,
v+w = 75 bis 200 und
n = 0 bis 3.

In einer bevorzugten Ausführungsform ist in der (Meth)acryl-Verbindung der Formel I X = O, Z = Y, n = 0 und w = 75 bis 180, bevorzugt 75 bis 150, besonders bevorzugt 100 bis 150.
In einer weiteren bevorzugten Ausführungsform ist in der (Meth)acryl-Verbindung der Formel I X = O, Z = Y, n = 1 bis 3, v = 36 bis 100, bevorzugt 36 bis 90, besonders bevorzugt 36 bis 80, w = 36 bis 100, bevorzugt 36 bis 90,

besonders bevorzugt 36 bis 80 und v+w = 75 bis 180, bevorzugt 75 bis 150, besonders bevorzugt 100 bis 150.

In einer weiteren bevorzugten Ausführungsform ist in der (Meth)acryl-Verbindung der Formel I X = O, Z = Y, n = 0 bis 3, v = 36 bis 100, bevorzugt 36 bis 90, besonders bevorzugt 36 bis 80, w = 36 bis 100, bevorzugt 36 bis 90, besonders bevorzugt 36 bis 80 und n*v+w = 75 bis 200, bevorzugt 75 bis 180, besonders bevorzugt 100 bis 150.

In einer bevorzugten Ausführungsform ist in der (Meth)acryl-Verbindung der Formel I $L^1$ eine geradkettige, verzweigte und/oder cyclische Alkylen-, Arylen-, Arylalkylen- oder Alkylarylengruppe, die -O-, -OC(=O)-, -C(=O)O-, -NHC(=O)-, -C(=O)NH-, - OC(=O)NH-, oder-NHC(=O)O- enthalten kann, und

$L^2$ eine geradkettige, verzweigte und/oder cyclische Alkylen-, Arylen-, Arylalkylen- oder Alkylarylengruppe, die -O-, -OC(=O)-, -C(=O)O-, -NHC(=O)-, -C(=O)NH-, - OC(=O)NH-, oder-NHC(=O)O- enthalten kann, vorzugsweise ist $L^1$ und/oder $L^2$ ausgewählt aus der Gruppe bestehend aus

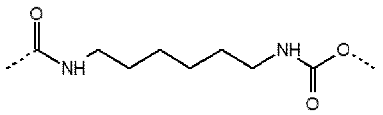 , 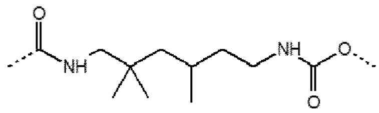 ,

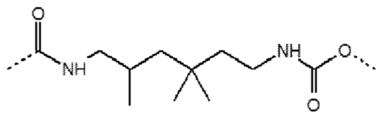 , 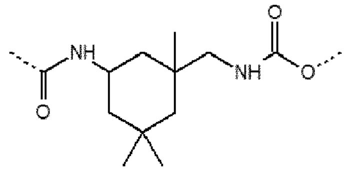 ,

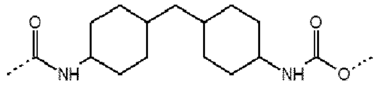 , 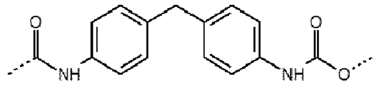 ,

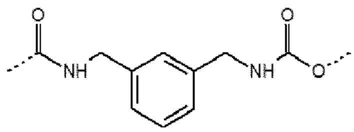 und 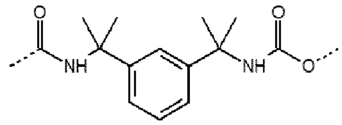 ,

bevorzugt ausgewählt aus der Gruppe bestehend aus

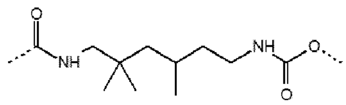 , 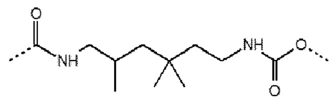 und 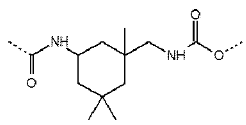 .

Dabei ist $L^1$ mit der linken gestrichelten Linie an das äußere Sauerstoffatom der Polypropylengruppe $(C_3H_6O)_v$ gebunden und mit der rechten gestrichelten Linie an das äußere Kohlenstoffatom der Polypropylengruppe $(C_3H_6O)_w$ gebunden und/oder $L^2$ ist mit der linken gestrichelten Linie an X gebunden und mit der rechten gestrichelten Linie über ein Sauerstoffatom ( -O- ) an das äußere Kohlenstoffatom einer der Polypropylengruppen $(C_3H_6O)_w$ oder $(C_3H_6O)_v$ gebunden.

In einer bevorzugten Ausführungsform weist die (Meth)acryl-Verbindung der Formel I ein Molekulargewicht von größer 5000 g/mol, bevorzugt von größer 6000 g/mol, besonders bevorzugt von größer 7000 g/mol, und/oder im Bereich von 5000 bis 15000 g/mol, bevorzugt im Bereich von 6000 bis 12000 g/mol, besonders bevorzugt im Bereich von 7000 bis 10000 g/mol, auf.

Monofunktionelle (Meth)acryl-Verbindung (A2)

Eine erfindungsgemäße Zusammensetzung enthält 10 bis 50 Gew.-%, bevorzugt 20 bis 50 Gew.-%, polymerisierbare monofunktionelle (Meth)acryl-Verbindungen (A2), bezogen auf die Gesamtmasse der polymerisierbaren Zusammensetzung.

In einer bevorzugten Ausführungsform handelt es sich um eine erfindungsgemäße Zusammensetzung, wobei die monofunktionelle (Meth)acryl-Verbindung (A2) ausgewählt ist aus der Gruppe bestehend aus 2-Hydroxyethyl(meth)acrylat, 2-Hydroxypropyl(meth)acrylat, 3-Hydroxypropyl(meth)acrylat, Tetrahydrofurfuryl(meth)acrylat, 4-(Meth)acryloylmorpholin, Cyclohexyl(meth)acrylat, 3,3,5-Trimethylcyclohexyl(meth)-acrylat, 4-(1,1-Dimethylethyl)cyclohexyl(meth)acrylat, Isobornyl(meth)acrylat, (Octahydro-4,7-methano-1H-indenyl)methyl(meth)acrylat, Benzyl(meth)acrylat, 2-Phenoxyethyl-(meth)acrylat, 2-(2-Phenoxyethoxy)ethyl(meth)acrylat, 2-[2-(2-Phenoxyethoxy)ethoxy]-ethyl(meth)acrylat, ethoxyliertes 2-Phenoxyethyl(meth)acrylat 2-Ethoxyethyl(meth)acrylat, 2-(2-Ethoxyethoxy)ethyl(meth)acrylat, 2-[2-(2-Ethoxyethoxy)ethoxy]ethyl(meth)acrylat, 2-Methoxyethyl(meth)acrylat, 2-(2-Methoxyethoxy)ethyl(meth)acrylat, 2-[2-(2-Methoxy-ethoxy)ethoxy]ethyl(meth)acrylat, Hexyl(meth)acrylat, 2-Ethylhexyl(meth)acrylat, Decyl(meth)acrylat, Isodecyl(meth)acrylat, Dodecyl(meth)acrylat, 2-Hydroxy-3-(prop-2-enoyloxy)propyl 2-methyl-2-propylhexanoat, und (5-Ethyl-1,3-dioxan-5-yl)methyl-(meth)acrylat.

Difunktionelle (Meth)acryl-Verbindung (A3)

Eine erfindungsgemäße Zusammensetzung enthält 1 bis 30 Gew.-%, bevorzugt 2 bis 15 Gew.-%, polymerisierbare difunktionelle (Meth)acryl-Verbindungen (A3), bezogen auf die Gesamtmasse der polymerisierbaren Zusammensetzung.

In einer bevorzugten Ausführungsform handelt es sich um eine erfindungsgemäße Zusammensetzung, wobei die difunktionelle (Meth)acryl-Verbindung (A3) ausgewählt ist aus der Gruppe bestehend aus Ethylenglycoldi(meth)acrylat, Diethylen-glycoldi(meth)acrylat, Triethylenglycoldi(meth)acrylat, Tetraethylenglycoldi(meth)acrylat, Polyethylenglycoldi(meth)acrylat, Propylenglycoldi(meth)acrylat, Dipropylenglycol-di(meth)acrylat, Tripropylenglycoldi(meth)acrylat, Tetrapropylenglycoldi(meth)acrylat, Polypropylenglycoldi(meth)acrylat, 1,4-Butandioldi(meth)acrylat, 1,3-Butandioldi(meth)-acrylat, 1,6-Hexandioldi(meth)acrylat, 1,10-Decandioldi(meth)acrylat, 1,12-Dodecandioldi-(meth)acrylat, 2-Hydroxypropyl-1,3-di(meth)acrylat, 3-Hydroxypropyl-1,2-di(meth)acrylat, Neopentylglycoldi(meth)acrylat, 2,2-Bis[4-[3-(meth)acryloyloxy-2-hydroxypropoxy]-phenyl]propan, 2,2-Bis[4-(meth)acryloyloxyphenyl]propan, 2,2-Bis[4-(meth)acryloyloxy-ethoxyphenyl]propan, 2,2-Bis[4-(meth)acryloyloxydiethoxyphenyl]propan, 2,2-Bis[4-(meth)acryloyloxytriethoxyphenyl]propan 2,2-Bis[4-(meth)acryloyloxytetraethoxyphenyl]-propan, 2,2-Bis[4-(meth)acryloyloxypentaethoxyphenyl]-propan, 2,2-Bis[4-(meth)acryl-oyloxydipropoxyphenyl]propan, 2,2-Bis[4-(meth)acryloyloxyethoxyphenyl]-2-[4-(meth)-acryloyloxydiethoxyphenyl]propan, 2-[4-(Meth)acryloyloxydiethoxyphenyl]-2-[4-(meth)-acryloyloxytriethoxyphenyl]propan, 2-[4-(Meth)acryloyloxydipropoxyphenyl]-2-[4-(meth)-acryloyloxytriethoxyphenyl]propan, 2,2-Bis[4-(meth)acryloyloxyisopropoxyphenyl]propan, ethoxyliertes Bisphenol-A-di(meth)acrylat, propoxyliertes Bisphenol-A-di(meth)acrylat, 3(4),8(9)-Bis((meth)acryloyloxymethyl)tricyclo[5.2.1.0$^{2,6}$]decan, 3,14-Dioxa-4,13-dioxo-5,12-diazahexadecan-1,16-diyldi(meth)¬acrylat, 7,7,9-Trimethyl-3,14-dioxa-4,13-dioxo-5,12-diazahexadecan-1,16-diyldi¬(meth)acrylat, 7,9,9-Trimethyl-3,14-dioxa-4,13-dioxo-5,12-diazahexadecan-1,16-diyldi(meth)acrylat, 1,5,5-Trimethyl-1-[(2-(meth)acryloyloxy-ethyl)-carbamoyl-methyl]-3-(2-(meth)acryloyloxyethyl)carbamoylcyclohexan (CAS 42405-01-6 Methacrylat; CAS 42404-50-2 Acrylat), 1,1'-[Methylenbis(4,1-phenylen-imino-carbonyloxy-2,1-ethandiyl)]bis(meth)acrylat (CAS 51243-61-9 Methacrylat; CAS 69790-08-5 Acrylat), 1,1'-[Methylenbis(2,1-phenyleniminocarbonyloxy-2,1-ethandiyl)]bis(meth)-acrylat.

Multifunktionelle (Meth)acryl-Verbindung (A4)

Eine erfindungsgemäße Zusammensetzung enthält 1 bis 20 Gew.-%, bevorzugt 1 bis 10 Gew.-%, polymerisierbare multifunktionelle (Meth)acryl-Verbindungen (A4), bezogen auf die Gesamtmasse der polymerisierbaren Zusammensetzung.

In einer bevorzugten Ausführungsform handelt es sich um eine erfindungsgemäße Zusammensetzung, wobei die multifunktionelle (Meth)acryl-Verbindung (A4) ausgewählt ist aus der Gruppe bestehend aus Glyceroltri(meth)acrylat, ethoxyliertes, Glyceroltri(meth)acrylat, propoxyliertes, Glyceroltri(meth)acrylat, Trimethylolethan-tri(meth)acrylat, ethoxyliertes Trimethylolethantri(meth)acrylat, Trimethylolpropan-tri(meth)acrylat, ethoxyliertes Trimethylolpropantri(meth)acrylat, Ditrimethylolpropan-tri(meth)acrylat, Ditrimethylolpropantetra(meth)acrylat, Pentaerythritoltri(meth)acrylat, Pentaerythritoltetra(meth)acrylat, ethoxyliertes Pentaerythritoltetra(meth)acrylat, Dipenta-erythritoltri(meth)acrylat, Dipentaerythritoltetra(meth)acrylat, Dipentaerythritolpenta-(meth)acrylat, Dipentaerythritolhexa(meth)acrylat, ethoxyliertes Dipentaerythritol-penta(meth)acrylat, ethoxyliertes Dipentaerythritolhexa(meth)acrylat und (2,4,6-Trioxo-1,3,5-triazin-1,3,5(2H,4H,6H)-triyl)tri-2,1 -ethandiyltri(meth)acrylat.

Photoinitiatoren (B)

Eine erfindungsgemäße Zusammensetzung enthält 0.1 bis 5 Gew.-%, bevorzugt 0.5 bis 4 Gew.-%, jeweils bezogen auf die Gesamtmasse der polymerisierbaren Zusammensetzung, eines Photoinitiators oder Photoinitiatorsystems (B) für die radikalische Polymerisation. Als Photoinitiatoren können die für (Meth)acryl-Systeme üblichen, geeigneten Verbindungen eingesetzt werden. Vorteilhaft werden alpha-Diketone, Benzoinalkylether, Thioxanthone, Benzophenone, Acetophenone, Acylphosphinoxide oder Acylgermanium-Verbindungen eingesetzt. Bevorzugt werden Monoacylphosphinoxide oder Bisacylphosphinoxide verwendet.

In einer bevorzugten Ausgestaltung ist (B) ausgewählt aus der Gruppe bestehend aus alpha-Diketonen, Benzoinalkylethern, Thioxanthonen, Benzophenonen, Acetophenonen, Acylphosphinoxiden, und Acylgermanium-Verbindungen, bevorzugt ausgewählt aus der Gruppe bestehend aus 1-Hydroxycyclohexylbenzophenon, 4-(2-Hydroxyethoxy)phenyl-(2-hydroxy-2-propyl)keton, 2-Hydroxy-2-methylpropiophenon, 2,4,6-Trimethylbenzoyldiphenylphosphinoxid, Phenyl-bis(2,4,6-trimethylbenzoyl)phosphinoxid, Ethyl phenyl(2,4,6-trimethylbenzoyl)phosphinate (CAS 84434-11-7) und (Poly(oxy-1,2-ethandiyl),α,α',α"-1,2,3-propantriyltris[ω-[[phenyl(2,4,6-trimethylbenzoyl)phosphinyl]oxy]-Polymer) (CAS 1834525-17-5), besonders bevorzugt ausgewählt aus der Gruppe bestehend aus 2,4,6-Trimethylbenzoyldiphenylphosphinoxid, Phenyl-bis(2,4,6-trimethylbenzoyl)phosphinoxid, Ethyl phenyl(2,4,6-trimethylbenzoyl)phosphinate und (Poly(oxy-1,2-ethandiyl),α,α',α"-1,2,3-propantriyltris[ω-[[phenyl(2,4,6-trimethylbenzoyl)phosphinyl]oxy]-Polymer).

Stabilisatoren / Inhibitoren (C)

In einer bevorzugten Ausführungsform enthält eine erfindungsgemäße Zusammensetzung 0.001 bis 1 Gew.-%, bevorzugt 0.01 bis 0.5 Gew.-%, besonders bevorzugt 0.01 bis 0.3 Gew.-%, jeweils bezogen auf die Gesamtmasse der polymerisierbaren Zusammensetzung, eines oder mehrerer Stabilisatoren (C).

Vorzugsweise ist (C) ausgewählt aus der Gruppe bestehend aus Hydrochinon, Hydrochinonmonomethylether, 2,6-Di-tert.-butyl-4-methylphenol, *tert*.-Butylhydroxyanisol und 2,2,6,6-Tetramethyl-piperidin-1-oxyl.

In einer bevorzugten Ausführungsform umfasst eine erfindungsgemäße Zusammensetzung

A1) in einer Menge von 30 bis 70 Gew.-%, bevorzugt 40 bis 60 Gew.-%,
A2) in einer Menge von 10 bis 50 Gew.-%, bevorzugt 20 bis 50 Gew.-%,
A3) in einer Menge von 1 bis 30 Gew.-%, bevorzugt 2 bis 15 Gew.-% und
B) in einer Menge von 0.1 bis 5 Gew.-%, bevorzugt 0.5 bis 4 Gew.-%, jeweils bezogen auf die Gesamtmasse der polymerisierbaren Zusammensetzung.

In einer weiteren bevorzugten Ausführungsform umfasst eine erfindungsgemäße Zusammensetzung

A1) in einer Menge von 30 bis 70 Gew.-%, bevorzugt 40 bis 60 Gew.-%,
A2) in einer Menge von 10 bis 50 Gew.-%, bevorzugt 20 bis 50 Gew.-%,
A3) in einer Menge von 1 bis 30 Gew.-%, bevorzugt 2 bis 15 Gew.-%,
A4) in einer Menge von 1 bis 20 Gew.-%, bevorzugt 1 bis 10 Gew.-% und
B) in einer Menge von 0.1 bis 5 Gew.-%, bevorzugt 0.5 bis 4 Gew.-%,

jeweils bezogen auf die Gesamtmasse der polymerisierbaren Zusammensetzung.

In einer weiteren bevorzugten Ausführungsform umfasst eine erfindungsgemäße Zusammensetzung

A1) in einer Menge von 30 bis 70 Gew.-%, bevorzugt 40 bis 60 Gew.-%,
A2) in einer Menge von 10 bis 50 Gew.-%, bevorzugt 20 bis 50 Gew.-%,
A3) in einer Menge von 1 bis 30 Gew.-%, bevorzugt 2 bis 15 Gew.-%,
A4) in einer Menge von 1 bis 20 Gew.-%, bevorzugt 1 bis 10 Gew.-%,
B) in einer Menge von 0.1 bis 5 Gew.-%, bevorzugt 0.5 bis 4 Gew.-% und
C) in einer Menge von 0.001 bis 1 Gew.-%, bevorzugt 0.01 bis 0.5 Gew.-%, jeweils bezogen auf die Gesamtmasse der polymerisierbaren Zusammensetzung.

Bevorzugt ist auch eine erfindungsgemäße Zusammensetzung, wobei die (Meth)acryl-Verbindung (A1) mindestens eine Urethangruppe enthält und/oder (vorzugsweise und) die (Meth)acryl-Verbindung (A2) keine Urethangruppe enthält und/oder (vorzugsweise und) die (Meth)acryl-Verbindung (A3) keine Urethangruppe enthält und/oder (vorzugsweise und) die (Meth)acryl-Verbindung (A4) keine Urethangruppe enthält.

Wie oben beschrieben resultiert aus dem Zusammenspiel der Monomere (A1), (A2), (A3) und ggf. (A4) ein Druckharz, das nach der Polymerisation keine nennenswerte Haftung zu anderen (Meth)acrylsystemen aufweist.

Erfindungsgemäß ist auch eine dentale, radikalisch polymerisierbare Zusammensetzung wie oben beschrieben, zur Verwendung in Stereolithographie und/oder 3D-Druck, vorzugsweise zum 3D-Druck von dentalen Formteilen, bevorzugt zum 3D-Druck von dentalen Transferschienen, IBTs, Übertragungsschlüsseln und Bohrschablonen.

Erfindungsgemäß ist auch die Verwendung einer dentalen, radikalisch polymerisierbaren Zusammensetzung wie oben beschrieben, in Stereolithographie und/oder 3D-Druck, vorzugsweise zum 3D-Druck von dentalen Formteilen, bevorzugt zum 3D-Druck von dentalen Transferschienen, IBTs, Übertragungsschlüsseln und Bohrschablonen.

Erfindungsgemäß ist auch eine dentale, radikalisch polymerisierbare Zusammensetzung wie oben beschrieben, zur Anwendung in einem Verfahren zur therapeutischen oder chirurgischen Behandlung des menschlichen Körpers, vorzugsweise zur spezifischen Anwendung in einem therapeutischen Verfahren zur Korrektur von Zahnfehlstellungen oder in einem chirurgischen Verfahren zum Bohren von Implantatlöchern.

Beispiele

Die Ziele und Vorteile dieser Offenbarung werden durch die folgenden Beispiele weiter veranschaulicht, aber die spezifischen Materialien und deren Mengen, die in diesen Beispielen genannt werden, sowie andere Bedingungen und Details sollten nicht so ausgelegt werden, dass sie diese Offenbarung unangemessen einschränken.

Die in den Beispielen verwendeten Materialien werden im Folgenden zusammengefasst.

| | |
|---|---|
| PPG | Polypropylengylcol (CAS 25322-69-4) |
| PEG | Polyethylengylcol (CAS 25322-68-3) |
| HMDI | Hexamethylendiisocyanat (CAS 822-06-0) |
| TMDI | 2,2,4-Trimethylhexamethylendiisocyanat / 2,4,4-Trimethylhexamethylendiisocyanat (Isomerengemisch; CAS 32052-51-0) |
| IPDI | Isophorondiisocyanat (CAS 4098-71-9) |
| DBTL | Dibutylzinndilaurat (CAS 77-58-7) |
| BHT | 2,6-Di-tert.-butyl-4-methylphenol (CAS 128-37-0) |
| IBOMA | Isobornylmethacrylat (CAS 7534-94-3) |
| ACMO | Acryloylmorpholin (CAS 5117-12-4) |
| THFMA | Tetrahydrofurfurylmethacrylat (CAS 2455-24-5) |
| PheMA | 2-Phenoxyethylmethacrylat (CAS 10595-06-9) |
| HEMA | 2-Hydroxyethylmethacrylat (CAS 868-77-9) |
| CTFA | (5-Ethyl-1,3-dioxan-5-yl)methylacrylat (CAS 66492-51-1) |
| BisEMA-6 | ethoxyliertes Bisphenol A Dimethacrylat (6 EO-Einheiten; CAS 41637-38-1) |
| TCDDMA | 3(4),8(9)-Bis(methacryloyloxymethyl)tricyclo[5.2.1.0$^{2,6}$]decan (CAS 43048-08-4) |
| HDDMA | 1,6-Hexandioldimethacrylat (CAS 6606-59-3) |
| UDMA | 7,7,9-Trimethyl-3,14-dioxa-4,13-dioxo-5,12-diazahexadecan-1,16-diyldi(meth)acrylat / 7,9,9-Trimethyl-3,14-dioxa-4,13-dioxo-5,12-diazahexadecan-1,16-diyldi(meth)acrylat (Isomerengemisch; CAS 72869-86-4) |
| PETA | Pentaerythritoltetraacrylat (CAS 4986-89-4) |
| PETA-4 | ethoxyliertes Pentaerythritoltetraacrylat (4 EO-Einheiten; CAS 51728-26-8) |
| TMPTA | Trimethylolpropantriacrylat (CAS 15625-89-5) |
| TMPTA-3 | ethoxyliertes Trimethylolpropantriacrylat (3 EO-Einheiten; CAS 28961-43-5) |
| ISCTA | (2,4,6-Trioxo-1,3,5-triazin-1,3,5(2H,4H,6H)-triyl)tri-2,1-ethandiyltriacrylat (CAS 40220-08-4) |
| TPO: | Diphenyl(2,4,6-trimethylbenzoyl)phosphinoxid (CAS 75980-60-8) |

Synthese der Polypropylenglycol(meth)acryl-Verbindungen

Beispiel 1a (PPG-5000-Dimethacrylat):

100.00 g PPG-5000 und 4.05 g Triethylamin werden in 400 ml Chloroform gelöst. 4.18 g Methacryloylchlorid werden in 20 ml Chloroform gelöst und bei 0 °C langsam zugetropft. Nach beendeter Zugabe wird noch eine Stunde bei 0 °C gerührt. Danach wird langsam auf Raumtemperatur erwärmt und für weitere 18 Stunden bei Raumtemperatur gerührt. Der gebildete Niederschlag wird abfiltriert und das Filtrat zweimal mit Wasser, dreimal mit 10%iger Salzsäure und zweimal mit gesättigter Kochsalzlösung gewaschen. Die organische Phase wird über Magnesiumsulfat getrocknet. Es werden 50 mg BHT zugegeben und das Lösungsmittel im Vakuum entfernt. Es wird eine schwach gelbe Flüssigkeit erhalten.

Beispiel 1b (PPG-6000-Dimethacrylat):

120.00 g PPG-6000 und 4.05 g Triethylamin werden in 400 ml Chloroform gelöst. 4.18 g Methacryloylchlorid werden in 25 ml Chloroform gelöst und bei 0 °C langsam zugetropft. Nach beendeter Zugabe wird noch eine Stunde

bei 0 °C gerührt. Danach wird langsam auf Raumtemperatur erwärmt und für weitere 18 Stunden bei Raumtemperatur gerührt. Der gebildete Niederschlag wird abfiltriert und das Filtrat zweimal mit Wasser, dreimal mit 10%iger Salzsäure und zweimal mit gesättigter Kochsalzlösung gewaschen. Die organische Phase wird über Magnesiumsulfat getrocknet. Es werden 50 mg BHT zugegeben und das Lösungsmittel im Vakuum entfernt. Es wird eine schwach gelbe Flüssigkeit erhalten.

Beispiel 1c (PPG-5000-Urethan-Dimethacrylat):

200.00 g PPG-5000 werden in 400 ml Chloroform gelöst. Anschließend werden 6.21 g 2-Isocyanatoethylmethacrylat, 200 mg Dibutylzinndilaurat und 100 mg BHT zugegeben. Es wird unter Rühren für 6 Stunden auf 55 °C erwärmt. Als Reaktionskontrolle wird das Verschwinden der NCO-Bande bei ca. 2270 $cm^{-1}$ im IR-Spektrum beobachtet. Anschließend wird das Lösungsmittel im Vakuum entfernt. Es wird eine schwach gelbe Flüssigkeit erhalten. Viskosität (25 °C): 0.5 Pa*s

Beispiel 1d (PPG-6000-Urethan-Dimethacrylat):

240.00 g PPG-6000 werden in 400 ml Chloroform gelöst. Anschließend werden 6.21 g 2-Isocyanatoethylmethacrylat, 200 mg Dibutylzinndilaurat und 100 mg BHT zugegeben. Es wird unter Rühren für 6 Stunden auf 55 °C erwärmt. Als Reaktionskontrolle wird das Verschwinden der NCO-Bande bei ca. 2270 $cm^{-1}$ im IR-Spektrum beobachtet. Anschließend wird das Lösungsmittel im Vakuum entfernt. Es wird eine schwach gelbe Flüssigkeit erhalten. Viskosität (25 °C): 0.5 Pa*s

Beispiel 1e (PPG-5000-HMDI-HEMA):

100.00 g PPG-5000, 6.73 g Hexamethylendiisocyanat (HMDI) und 5.21 g 2-Hydroxyethylmethacrylat (HEMA) werden in 200 ml Chloroform gelöst. Anschließend werden 100 mg Dibutylzinndilaurat und 50 mg BHT zugegeben. Es wird unter Rühren für 6 Stunden auf 55 °C erwärmt. Als Reaktionskontrolle wird das Verschwinden der NCO-Bande bei ca. 2270 $cm^{-1}$ im IR-Spektrum beobachtet. Anschließend wird das Lösungsmittel im Vakuum entfernt. Es wird eine schwach gelbe Flüssigkeit erhalten.

Beispiel 1f (PPG-5000-TMDI-HEA):

100.00 g PPG-5000, 8.41 g Trimethylhexamethylendiisocyanat (TMDI) und 4.64 g 2-Hydroxyethylacrylat (HEA) werden in 200 ml Chloroform gelöst. Anschließend werden 100 mg Dibutylzinndilaurat und 50 mg BHT zugegeben. Es wird unter Rühren für 6 Stunden auf 55 °C erwärmt. Als Reaktionskontrolle wird das Verschwinden der NCO-Bande bei ca. 2270 $cm^{-1}$ im IR-Spektrum beobachtet. Anschließend wird das Lösungsmittel im Vakuum entfernt. Es wird eine schwach gelbe Flüssigkeit erhalten.

Beispiel 1g (PPG-5000-IPDI-HEMA):

100.00 g PPG-5000, 8.89 g Isophorondiisocyanat (IPDI) und 5.21 g 2-Hydroxyethylmethacrylat (HEMA) werden in 200 ml Chloroform gelöst. Anschließend werden 100 mg Dibutylzinndilaurat und 50 mg BHT zugegeben. Es wird unter Rühren für 6 Stunden auf 55 °C erwärmt. Als Reaktionskontrolle wird das Verschwinden der NCO-Bande bei ca. 2270 $cm^{-1}$ im IR-Spektrum beobachtet. Anschließend wird das Lösungsmittel im Vakuum entfernt. Es wird eine schwach gelbe Flüssigkeit erhalten.

Beispiel 1h (PPG-5000-IPDI-HEA):

100.00 g PPG-5000, 8.89 g Isophorondiisocyanat (IPDI) und 4.64 g 2-Hydroxyethylacrylat (HEA) werden in 200 ml Chloroform gelöst. Anschließend werden 100 mg Dibutylzinndilaurat und 50 mg BHT zugegeben. Es wird unter Rühren für 6 Stunden auf 55 °C erwärmt. Als Reaktionskontrolle wird das Verschwinden der NCO-Bande bei ca. 2270 $cm^{-1}$ im IR-Spektrum beobachtet. Anschließend wird das Lösungsmittel im Vakuum entfernt. Es wird eine schwach gelbe Flüssigkeit erhalten.

Beispiel 1i (oligo-PPG-5000-IPDI-HEMA):

100.00 g PPG-3000, 11.12 g Isophorondiisocyanat (IPDI) und 4.38 g 2-Hydroxyethylmethacrylat (HEMA) werden in 200 ml Chloroform gelöst. Anschließend werden 100 mg Dibutylzinndilaurat und 50 mg BHT zugegeben. Es wird

unter Rühren für 6 Stunden auf 55 °C erwärmt. Als Reaktionskontrolle wird das Verschwinden der NCO-Bande bei ca. 2270 $cm^{-1}$ im IR-Spektrum beobachtet. Anschließend wird das Lösungsmittel im Vakuum entfernt. Es wird eine schwach gelbe Flüssigkeit erhalten.

Vergleichsbeispiel V1a (PPG-2000-Dimethacrylat):

40.00 g PPG-2000 und 4.05 g Triethylamin werden in 200 ml Chloroform gelöst. 4.18 g Methacryloylchlorid werden in 25 ml Chloroform gelöst und bei 0 °C langsam zugetropft. Nach beendeter Zugabe wird noch eine Stunde bei 0 °C gerührt. Danach wird langsam auf Raumtemperatur erwärmt und für weitere 18 Stunden bei Raumtemperatur gerührt. Der gebildete Niederschlag wird abfiltriert und das Filtrat zweimal mit Wasser, dreimal mit 10%iger Salzsäure und zweimal mit gesättigter Kochsalzlösung gewaschen. Die organische Phase wird über Magnesiumsulfat getrocknet. Es werden 50 mg BHT zugegeben und das Lösungsmittel im Vakuum entfernt. Es wird eine schwach gelbe Flüssigkeit erhalten.

Vergleichsbeispiel V1b (PPG-1000-Urethan-Dimethacrylat):

80.00 g PPG-1000 werden in 200 ml Chloroform gelöst. Anschließend werden 12.42 g 2-Isocyanatoethylmethacrylat, 200 mg Dibutylzinndilaurat und 100 mg BHT zugegeben. Es wird unter Rühren für 6 Stunden auf 55 °C erwärmt. Als Reaktionskontrolle wird das Verschwinden der NCO-Bande bei ca. 2270 $cm^{-1}$ im IR-Spektrum beobachtet. Anschließend wird das Lösungsmittel im Vakuum entfernt. Es wird eine schwach gelbe Flüssigkeit erhalten. Viskosität (25 °C): 1.3 Pa*s

Vergleichsbeispiel V1c (PPG-4000-Urethan-Dimethacrylat):

160.00 g PPG-4000 werden in 400 ml Chloroform gelöst. Anschließend werden 12.42 g 2-Isocyanatoethylmethacrylat, 200 mg Dibutylzinndilaurat und 100 mg BHT zugegeben. Es wird unter Rühren für 6 Stunden auf 55 °C erwärmt. Als Reaktionskontrolle wird das Verschwinden der NCO-Bande bei ca. 2270 $cm^{-1}$ im IR-Spektrum beobachtet. Anschließend wird das Lösungsmittel im Vakuum entfernt. Es wird eine schwach gelbe Flüssigkeit erhalten. Viskosität (25 °C): 0.5 Pa*s

Vergleichsbeispiel V1d (PPG-1000-TMDI-HEMA):

40.00 g PPG-1000, 16.82 g Trimethylhexamethylendiisocyanat (TMDI) und 9.29 g 2-Hydroxyethylacrylat (HEA) werden in 200 ml Chloroform gelöst. Anschließend werden 100 mg Dibutylzinndilaurat und 50 mg BHT zugegeben. Es wird unter Rühren für 6 Stunden auf 55 °C erwärmt. Als Reaktionskontrolle wird das Verschwinden der NCO-Bande bei ca. 2270 $cm^{-1}$ im IR-Spektrum beobachtet. Anschließend wird das Lösungsmittel im Vakuum entfernt. Es wird eine schwach gelbe Flüssigkeit erhalten.

Beispiel V1e (PEG-6000-Dimethacrylat):

120.00 g PEG-6000 und 4.05 g Triethylamin werden in 400 ml Chloroform gelöst. 4.18 g Methacryloylchlorid werden in 25 ml Chloroform gelöst und bei 0 °C langsam zugetropft. Nach beendeter Zugabe wird noch eine Stunde bei 0 °C gerührt. Danach wird langsam auf Raumtemperatur erwärmt und für weitere 18 Stunden bei Raumtemperatur gerührt. Der gebildete Niederschlag wird abfiltriert und das Filtrat zweimal mit Wasser, dreimal mit 10%iger Salzsäure und zweimal mit gesättigter Kochsalzlösung gewaschen. Die organische Phase wird über Magnesiumsulfat getrocknet. Es werden 50 mg BHT zugegeben und das Lösungsmittel im Vakuum entfernt. Es wird eine schwach gelbe Flüssigkeit erhalten.

Messmethoden:

Biegefestigkeit (BF): Die Biegefestigkeiten wurden angepasst an ISO 4049:2009 bestimmt. Aus den Druckharzen wurden mittels 3D-Druck (SolFlex350, W2P Engineering GmbH; Wellenlänge 385 nm, Leistung 8.3 $mW/cm^2$, Pixelgröße 50 $\mu m$, Schichtstärke 50 $\mu m$) Prüfkörper mit der Abmessung 40 mm x 5 mm x 5 mm hergestellt und mit einem Otoflash (VOCO GmbH) mit 2x2000 Blitzen nachbelichtet. Die Bestimmung der Biegefestigkeit erfolgt bei einer Vorschubgeschwindigkeit von 1 mm/min an einer Zwick-Universalprüfmaschine (Zwick GmbH & Co. KG, Ulm).

Haftung (HF): Für die Bestimmung der Haftung wurden aus den Druckharzen mittels 3D-Druck (SolFlex350, W2P Engineering GmbH; Wellenlänge 385 nm, Leistung 8.3 $mW/cm^2$, Pixelgröße 50 $\mu m$, Schichtstärke 50 $\mu m$) Prüfkörper mit einem Durchmesser von 20 mm und einer Höhe von 10 mm hergestellt und mit einem Otoflash (VOCO GmbH)

mit 2x2000 Blitzen nachbelichtet. Die Oberfläche wurde mit Isopropanol gereinigt. Anschließend wird ein Silikonring (Innendurchmesser 5 mm, Höhe 5 mm) aufgelegt und mit Structur 2 SC (VOCO GmbH) gefüllt. Die Aushärtung erfolgt für 24 Stunden bei 37 °C und 100% Luftfeuchtigkeit. Anschließend wird der Silikonring entfernt und es findet zunächst eine subjektive Prüfung der Haftung statt. Prüfkörper, bei denen sich kein Verbund zwischen gedrucktem Prüfkörper und Structur 2 SC gebildet hat werden mit "keine Haftung" bewertet und es wird ein Haftwert von 0 MPa angesetzt. Bei Prüfkörpern, bei denen sich ein Verbund zwischen gedrucktem Prüfkörper und Structur 2 SC gebildet hat werden zur Bestimmung des Haftwerts an einer Universalprüfmaschine (Zwick GmbH & Co. KG, Ulm) bei einer Vorschubgeschwindigkeit von 1 mm/min bis zum Bruch belastet. Der Haftwert ergibt sich als Quotient der Bruchkraft (in N) und der Haftfläche (in $mm^2$) jeweils als Mittelwert aus sechs Einzelmessungen.

Viskosität: Gemessen wurde standardmäßig an einem Rheometer der Firma Anton Paar des Typs Physica MCR 301 mit einer 50 mm-Messplatte (Platte/Platte), 0.5 mm Spaltabstand und 1 g Substanz. Vor der Messung wird die Platte auf eine Temperatur von 25 °C temperiert. Die Messdauer beträgt 30 s bei einer Scherrate von 10/s.

Molekulargewicht: Das Molekulargewicht wurde mit Hilfe einer GPC (Agilent Technologies 1260 Infinity) bestimmt. Das System besteht aus einer Vorsäule (Länge 50 mm, Durchmesser 8 mm, Partikelgröße 5 µm) und zwei Hauptsäulen (I: Länge 300 mm, Durchmesser 8 mm, Partikelgröße 5 µm, 50 A; II: Länge 300 mm, Durchmesser 8 mm, Partikelgröße 5 µm, 100 A). Als Laufmittel wird THF verwendet. Als Standard wird Polystyrol verwendet.

Ausführungsbeispiele:

Die in den Tabellen 1 bis 6 aufgeführten Bestandteile wurden jeweils in den angegebenen Mengen eingewogen und mit einem KPG-Rührwerk für 60 Minuten bei Raumtemperatur gerührt, bis eine homogene Lösung entstanden war.

Tabelle 1:

| Beispiel | | 2a | 2b | 2c | 2d | 2e | 2f |
|---|---|---|---|---|---|---|---|
| (A1) | Monomer 1a | 50.00 | 50.00 | 50.00 | 50.00 | 50.00 | 50.00 |
| (A2) | IBOMA | 30.00 | - | - | - | - | - |
| | ACMO | - | 30.00 | - | - | - | - |
| | THFMA | - | - | 30.00 | - | - | - |
| | PheMA | - | - | - | 30.00 | - | - |
| | HEMA | - | - | - | - | 30.00 | - |
| | CTFA | - | - | - | - | - | 30.00 |
| (A3) | BisEMA-6 | 10.00 | 10.00 | 10.00 | 10.00 | 10.00 | 10.00 |
| (A4) | PETA | 8.50 | 8.50 | 8.50 | 8.50 | 8.50 | 8.50 |
| (B) | TPO | 1.50 | 1.50 | 1.50 | 1.50 | 1.50 | 1.50 |
| Haftung [MPa] | | 0* | 0* | 0* | 0* | 0* | 0* |
| * zwischen gedrucktem Prüfkörper und Structur 2 SC hatte sich kein Verbund gebildet, so dass eine Messung der Haftwerte nicht möglich war. | | | | | | | |

Tabelle 2:

| Beispiel | | 2g | 2h | 2i | 2j | 2k | 2l |
|---|---|---|---|---|---|---|---|
| (A1) | Monomer 1a | 50.00 | 50.00 | 50.00 | 50.00 | 55.00 | 45.00 |
| (A2) | IBOMA | 30.00 | 30.00 | 30.00 | 30.00 | 20.00 | 40.00 |
| (A3) | BisEMA-6 | 10.00 | - | - | | 15.00 | 5.00 |
| | TCDDMA | - | 10.00 | - | | - | - |
| | HDDMA | - | - | 10.00 | - | - | - |
| | UDMA | - | - | - | 10.00 | - | - |
| (A4) | PETA | 8.50 | 8.50 | 8.50 | 8.50 | 8.50 | 8.50 |

(fortgesetzt)

| Beispiel | | 2g | 2h | 2i | 2j | 2k | 2l |
|---|---|---|---|---|---|---|---|
| (B) | TPO | 1.50 | 1.50 | 1.50 | 1.50 | 1.50 | 1.50 |
| Haftung [MPa] | | 0* | 0* | 0* | 0* | 0* | 3.3** |
| * zwischen gedrucktem Prüfkörper und Structur 2 SC hatte sich kein Verbund gebildet, so dass eine Messung der Haftwerte nicht möglich war.<br>** bei 4 von 6 Prüfkörpern hatte sich kein Verbund gebildet. | | | | | | | |

Tabelle 3:

| Beispiel | | 2m | 2n | 2o | 2p | 2q | 2r |
|---|---|---|---|---|---|---|---|
| (A1) | Monomer 1a | 50.00 | 50.00 | 50.00 | 50.00 | 50.00 | 50.00 |
| (A2) | IBOMA | 30.00 | 30.00 | 30.00 | 30.00 | 30.00 | 30.00 |
| (A3) | BisEMA-6 | 10.00 | 10.00 | 10.00 | 10.00 | 15.00 | 8.50 |
| (A4) | PETA | - | - | - | - | 3.50 | 10.00 |
| | PETA-4 | 8.50 | - | - | - | - | - |
| | TMPTA | - | 8.50 | - | - | - | - |
| | TMPTA-3 | - | - | 8.50 | - | - | - |
| | ISCTA | - | - | - | 8.50 | - | - |
| (B) | TPO | 1.50 | 1.50 | 1.50 | 1.50 | 1.50 | 1.50 |
| Haftung [MPa] | | 0* | 0* | 0* | 0* | 0* | 0* |
| * zwischen gedrucktem Prüfkörper und Structur 2 SC hatte sich kein Verbund gebildet, so dass eine Messung der Haftwerte nicht möglich war. | | | | | | | |

Tabelle 4:

| Beispiel | | 2s | 2t | 2u | 2v | 2w | 2x |
|---|---|---|---|---|---|---|---|
| (A1) | Monomer 1b | 50.00 | - | - | - | - | - |
| | Monomer 1c | - | 50.00 | - | - | - | - |
| | Monomer 1d | - | - | 50.00 | - | - | - |
| | Monomer 1e | - | - | - | 50.00 | - | - |
| | Monomer 1f | - | - | - | - | 50.00 | - |
| | Monomer 1g | - | - | - | - | - | 50.00 |
| (A2) | IBOMA | 30.00 | 30.00 | 30.00 | 30.00 | 30.00 | 30.00 |
| (A3) | BisEMA-6 | 10.00 | 10.00 | 10.00 | 10.00 | 10.00 | 10.00 |
| (A4) | PETA | 8.50 | 8.50 | 8.50 | 8.50 | 8.50 | 8.50 |
| (B) | TPO | 1.50 | 1.50 | 1.50 | 1.50 | 1.50 | 1.50 |
| Haftung [MPa] | | 0* | 0* | 0* | 0* | 0* | 0* |
| * zwischen gedrucktem Prüfkörper und Structur 2 SC hatte sich kein Verbund gebildet, so dass eine Messung der Haftwerte nicht möglich war. | | | | | | | |

Tabelle 5:

| Vergleichsbeispiel | | 3a | 3b | 3c | 3d | 3e | 3f |
|---|---|---|---|---|---|---|---|
| (A1) | Monomer 1a | 50.00 | 50.00 | 50.00 | - | 10.00 | 80.00 |
| (A2) | IBOMA | - | 40.00 | 30.00 | 60.00 | 55.00 | 15.00 |
| (A3) | BisEMA-6 | 40.00 | - | 18.50 | 20.00 | 18.50 | 2.00 |
| (A4) | PETA | 8.50 | 8.50 | - | 18.50 | 15.00 | 1.50 |
| (B) | TPO | 1.50 | 1.50 | 1.50 | 1.50 | 1.50 | 1.50 |
| Haftung [MPa] | | 35.7 | 21.8 | 37.4 | 33.1 | 19.4 | 29.7 |

Tabelle 6:

| Vergleichsbeispiel | | 3g | 3h | 3i | 3j | 3k |
|---|---|---|---|---|---|---|
| | Monomer V1a | 50.00 | - | - | - | - |
| | Monomer V1b | - | 50.00 | - | - | - |
| | Monomer V1c | - | - | 50.00 | - | - |
| | Monomer V1d | - | - | - | 50.00 | - |
| | Monomer V1e | - | - | - | - | 50.00 |
| (A2) | IBOMA | 30.00 | 30.00 | 30.00 | 30.00 | 30.00 |
| (A3) | BisEMA-6 | 10.00 | 10.00 | 10.00 | 10.00 | 10.00 |
| (A4) | PETA | 8.50 | 8.50 | 8.50 | 8.50 | 8.50 |
| (B) | TPO | 1.50 | 1.50 | 1.50 | 1.50 | 1.50 |
| Haftung [MPa] | | 25.4 | 20.3 | 12.3 | 14.6 | 29.1 |

Insgesamt lässt sich die Erfindung anhand der folgenden Aspekte zusammenfassen.

1. Dentale, radikalisch polymerisierbare Zusammensetzung umfassend

A1) eine oder mehrere polymerisierbare (Meth)acryl-Verbindungen der Formel I

$$Y\text{-}O\text{-}[(C_3H_6O)_v\text{-}L^1]_n\text{-}(C_3H_6O)_w\text{-}Z \qquad (I)$$

wobei

$Y = H_2C{=}C(R^1)\text{-}C({=}O)\text{-}$ oder $H_2C{=}C(R^1)\text{-}C({=}O)\text{-}X\text{-}L^2\text{-}$,
$R^1$ = H oder $CH_3$,
X = O oder NH,
$L^1$ = divalente organische Linkergruppe,
$L^2$ = divalente organische Linkergruppe,
Z = Y, H oder C1 bis C4-Alkyl,
v = 36 bis 100,
w = 36 bis 200,
n = 0 bis 3,
n*v+w = 36 bis 200

A2) eine oder mehrere monofunktionelle (Meth)acryl-Verbindungen mit einer (Meth)acryl-Gruppe, die nicht der Formel I entsprechen,
A3) eine oder mehrere difunktionelle (Meth)acryl-Verbindungen mit zwei (Meth)acryl-Gruppen, die nicht der Formel I entsprechen,
B) einen Photoinitiator oder ein Photoinitiatorsystem.

2. Dentale, radikalisch polymerisierbare Zusammensetzung nach Aspekt 1, zusätzlich umfassend A4) eine oder mehrere multifunktionelle (Meth)acryl-Verbindungen mit drei bis sechs (Meth)acryl-Gruppen.

3. Dentale, radikalisch polymerisierbare Zusammensetzung nach Aspekt 1, umfassend (A1) in einer Menge von 30 bis 70 Gew.-%, bevorzugt 40 bis 60 Gew.-%, bezogen auf die Gesamtmasse der polymerisierbaren Zusammensetzung.

4. Dentale, radikalisch polymerisierbare Zusammensetzung nach einem der vorangehenden Aspekte, wobei in der (Meth)acryl-Verbindung der Formel I X = O, Z = Y, n = 0 und w = 75 bis 180, bevorzugt 75 bis 150, besonders bevorzugt 100 bis 150.

5. Dentale, radikalisch polymerisierbare Zusammensetzung nach einem der vorangehenden Aspekte, wobei in der (Meth)acryl-Verbindung der Formel I X = O, Z = Y, n = 1 bis 3, v = 36 bis 100, bevorzugt 36 bis 90, besonders bevorzugt 36 bis 80, w = 36 bis 100, bevorzugt 36 bis 90, besonders bevorzugt 36 bis 80 und v+w = 75 bis 180, bevorzugt 75 bis 150, besonders bevorzugt 100 bis 150.

6. Dentale, radikalisch polymerisierbare Zusammensetzung nach einem der vorangehenden Aspekte, wobei in der (Meth)acryl-Verbindung der Formel I X = O, Z = Y, n = 0 bis 3, v = 36 bis 100, bevorzugt 36 bis 90, besonders bevorzugt 36 bis 80, w = 36 bis 100, bevorzugt 36 bis 90, besonders bevorzugt 36 bis 80 und n*v+w = 75 bis 200, bevorzugt 75 bis 180, besonders bevorzugt 100 bis 150.

7. Dentale, radikalisch polymerisierbare Zusammensetzung nach einem der vorangehenden Aspekte, wobei in der (Meth)acryl-Verbindung der Formel I $L^1$ eine geradkettige, verzweigte und/oder cyclische Alkylen-, Arylen-, Arylalkylen- oder Alkylarylengruppe, die -O-, -OC(=O)-, -C(=O)O-, -NHC(=O)-, -C(=O)NH-, - OC(=O)NH-, oder-NHC(=O)O- enthalten kann ist, und
$L^2$ eine geradkettige, verzweigte und/oder cyclische Alkylen-, Arylen-, Arylalkylen- oder Alkylarylengruppe, die -O-, -OC(=O)-, -C(=O)O-, -NHC(=O)-, -C(=O)NH-, - OC(=O)NH-, oder-NHC(=O)O- enthalten kann ist.

8. Dentale, radikalisch polymerisierbare Zusammensetzung nach einem der vorangehenden Aspekte, wobei $L^1$ und/oder $L^2$ ausgewählt sind aus der Gruppe bestehend aus

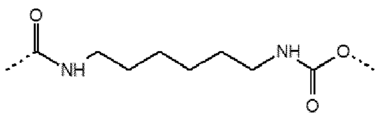 , 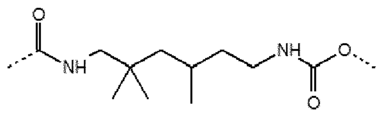 ,

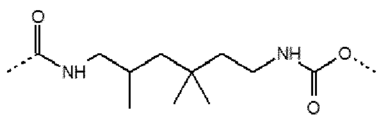 , 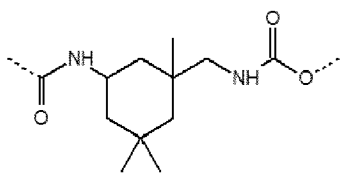 ,

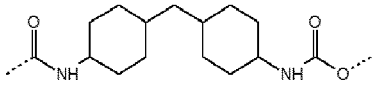 , 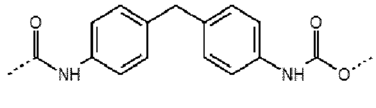 ,

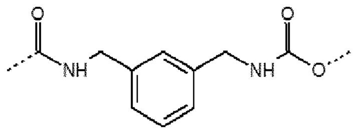 und 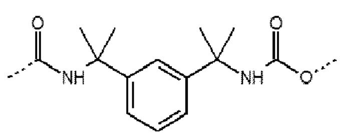 ,

bevorzugt ausgewählt aus der Gruppe bestehend aus

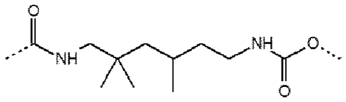 , 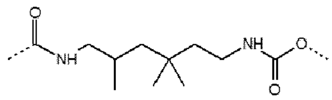

und

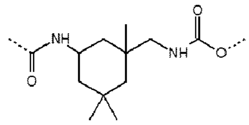

.

9. Dentale, radikalisch polymerisierbare Zusammensetzung nach einem der vorangehenden Aspekte, wobei $L^1$ und/oder $L^2$ ausgewählt sind aus der Gruppe bestehend aus

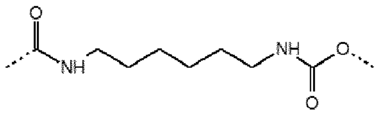 , 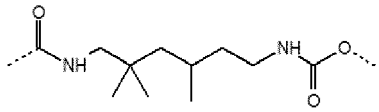 ,

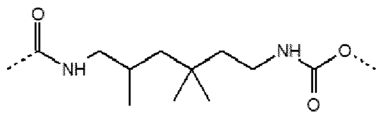 , 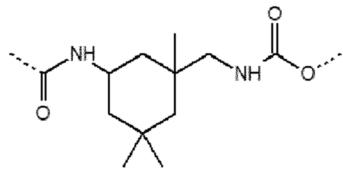 ,

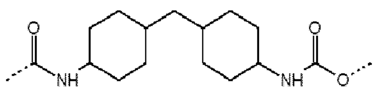 , 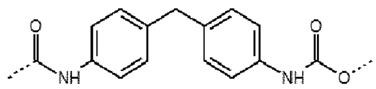 ,

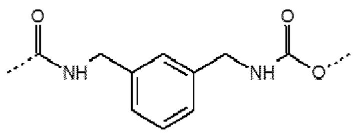 und 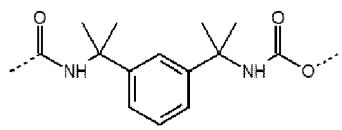 ,

bevorzugt ausgewählt aus der Gruppe bestehend aus

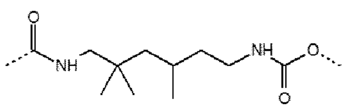 , 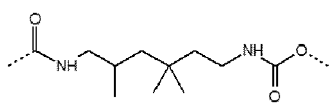

und

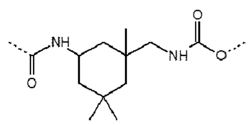

,

wobei $L^1$ mit der linken gestrichelten Linie an das äußere Sauerstoffatom der Polypropylengruppe $(C_3H_6O)_v$ gebunden ist und mit der rechten gestrichelten Linie an das äußere Kohlenstoffatom der Polypropylengruppe $(C_3H_6O)_w$ gebunden ist und/oder $L^2$ mit der linken gestrichelten Linie an X gebunden ist und mit der rechten gestrichelten

Linie über ein Sauerstoffatom ( -O- ) an das äußere Kohlenstoffatom einer der Polypropylengruppen $(C_3H_6O)_w$ oder $(C_3H_6O)_v$ gebunden ist.

10. Dentale, radikalisch polymerisierbare Zusammensetzung nach einem der vorangehenden Aspekte, wobei die (Meth)acryl-Verbindung der Formel I ein Molekulargewicht von größer 5000 g/mol, bevorzugt von größer 6000 g/mol, besonders bevorzugt von größer 7000 g/mol, und/oder im Bereich von 5000 bis 15000 g/mol, bevorzugt im Bereich von 6000 bis 12000 g/mol, besonders bevorzugt im Bereich von 7000 bis 10000 g/mol, aufweist.

11. Dentale, radikalisch polymerisierbare Zusammensetzung nach einem der vorangehenden Aspekte, umfassend (A2) in einer Menge von 10 bis 50 Gew.-%, bevorzugt 20 bis 50 Gew.-%, bezogen auf die Gesamtmasse der polymerisierbaren Zusammensetzung.

12. Dentale, radikalisch polymerisierbare Zusammensetzung nach einem der vorangehenden Aspekte, wobei die monofunktionelle (Meth)acryl-Verbindung (A2) ausgewählt ist aus der Gruppe bestehend aus 2-Hydroxyethyl(meth)acrylat, 2-Hydroxypropyl(meth)acrylat, 3-Hydroxypropyl(meth)acrylat, Tetrahydrofurfuryl-(meth)acrylat, 4-(Meth)acryloylmorpholin, Cyclohexyl(meth)acrylat, 3,3,5-Trimethylcyclohexyl(meth)acrylat, 4-(1,1-Dimethylethyl)cyclohexyl(meth)acrylat, Isobornyl(meth)acrylat, (Octahydro-4,7-methano-1H-indenyl)methyl(meth)acrylat, Benzyl(meth)acrylat, 2-Phenoxyethyl(meth)acrylat, 2-(2-Phenoxyethoxy)-ethyl(meth)acrylat, 2-[2-(2-Phenoxyethoxy)ethoxy]ethyl(meth)acrylat, ethoxyliertes 2-Phenoxyethyl(meth)acrylat 2-Ethoxyethyl(meth)acrylat, 2-(2-Ethoxyethoxy)ethyl-(meth)acrylat, 2-[2-(2-Ethoxyethoxy)ethoxy]ethyl(meth)acrylat, 2-Methoxyethyl-(meth)acrylat, 2-(2-Methoxyethoxy)ethyl(meth)acrylat, 2-[2-(2-Methoxyethoxy)-ethoxy]ethyl(meth)acrylat, Hexyl(meth)acrylat, 2-Ethylhexyl(meth)acrylat, Decyl-(meth)acrylat, Isodecyl(meth)acrylat, Dodecyl(meth)acrylat, 2-Hydroxy-3-(prop-2-enoyloxy)propyl 2-methyl-2-propylhexanoat, und (5-Ethyl-1,3-dioxan-5-yl)-methyl(meth)acrylat.

13. Dentale, radikalisch polymerisierbare Zusammensetzung nach einem der vorangehenden Aspekte, umfassend (A3) in einer Menge von 1 bis 30 Gew.-%, bevorzugt 2 bis 15 Gew.-%, bezogen auf die Gesamtmasse der polymerisierbaren Zusammensetzung.

14. Dentale, radikalisch polymerisierbare Zusammensetzung nach einem der vorangehenden Aspekte, wobei die difunktionelle (Meth)acryl-Verbindung (A3) ausgewählt ist aus der Gruppe bestehend aus Ethylenglycoldi(meth)acrylat, Diethylenglycoldi(meth)acrylat, Triethylenglycoldi(meth)acrylat, Tetraethylenglycol-di(meth)acrylat, Polyethylenglycoldi(meth)acrylat, Propylenglycoldi(meth)acrylat, Dipropylenglycoldi(meth)acrylat, Tripropylenglycoldi(meth)acrylat, Tetrapropylen-glycoldi(meth)acrylat, Polypropylenglycoldi(meth)acrylat, 1,4-Butandioldi(meth)-acrylat, 1,3-Butandioldi(meth)acrylat, 1,6-Hexandioldi(meth)acrylat, 1,10-Decan-dioldi(meth)acrylat, 1,12-Dodecandioldi(meth)acrylat, 2-Hydroxypropyl-1,3-di(meth)acrylat, 3-Hydroxypropyl-1,2-di(meth)acrylat, Neopentylglycoldi(meth)acrylat, 2,2-Bis[4-[3-(meth)acryloyloxy-2-hydroxypropoxy]phenyl]propan, 2,2-Bis[4-(meth)-acryloyloxyphenyl]propan, 2,2-Bis[4-(meth)acryloyloxyethoxyphenyl]propan, 2,2-Bis[4-(meth)acryloyloxydiethoxyphenyl]propan, 2,2-Bis[4-(meth)acryloyloxytriethoxy-phenyl]propan 2,2-Bis[4-(meth)acryloyloxytetraethoxyphenyl]propan, 2,2-Bis[4-(meth)acryloyloxypentaethoxyphenyl]-propan, 2,2-Bis[4-(meth)acryloyloxydipropoxy-phenyl]propan, 2,2-Bis[4-(meth)acryloyloxyethoxyphenyl]-2-[4-(meth)acryloyloxy-diethoxyphenyl]propan, 2-[4-(Meth)acryloyloxydiethoxyphenyl]-2-[4-(meth)-acryloyl-oxytriethoxyphenyl]propan, 2-[4-(Meth)acryloyloxydipropoxyphenyl]-2-[4-(meth)acryl-oyloxytriethoxyphenyl]propan, 2,2-Bis[4-(meth)acryloyloxyisopropoxyphenyl]propan, ethoxyliertes Bisphenol-A-di(meth)acrylat, propoxyliertes Bisphenol-A-di(meth)-acrylat, 3(4),8(9)-Bis((meth)acryloyloxymethyl)tricyclo[5.2.1.$0^{2,6}$]decan, 3,14-Dioxa-4,13-dioxo-5,12-diazahexadecan-1,16-diyldi(meth)¬acrylat, 7,7,9-Trimethyl-3,14-dioxa-4,13-dioxo-5,12-diazahexadecan-1,16-diyldi¬(meth)acrylat, 7,9,9-Trimethyl-3,14-dioxa-4,13-dioxo-5,12-diazahexadecan-1,16-diyldi(meth)acrylat, 1,5,5-Trimethyl-1-[(2-(meth)acryloyloxyethyl)¬carbamoyl-methyl]-3-(2-(meth)acryloyloxy-ethyl)carbamoylcyclohexan (CAS 42405-01-6 Methacrylat; CAS 42404-50-2 Acrylat), 1,1'-[Methylenbis(4,1-phenylen¬imino-carbonyloxy-2,1-ethandiyl)]bis(meth)acrylat (CAS 51243-61-9 Methacrylat; CAS 69790-08-5 Acrylat), 1,1'-[Methylenbis(2,1-phenyleniminocarbonyloxy-2,1-ethandiyl)]bis(meth)acrylat.

15. Dentale, radikalisch polymerisierbare Zusammensetzung nach einem der vorangehenden Aspekte, wobei die difunktionelle (Meth)acryl-Verbindung (A3) ausgewählt ist aus der Gruppe bestehend aus Ethylenglycoldi(meth)acrylat, Diethylenglycoldi(meth)acrylat, Triethylenglycoldi(meth)acrylat, Tetraethylenglycoldi(meth)acrylat, Polyethylenglycoldi(meth)acrylat, Propylenglycoldi(meth)acrylat, Dipropylenglycoldi(meth)acrylat, Tripropylenglycoldi(meth)acrylat, Tetrapropylen-glycoldi(meth)acrylat, Polypropylenglycoldi(meth)acrylat, 1,4-Butandioldi(meth)-acrylat, 1,3-Butandioldi(meth)acrylat, 1,6-Hexandioldi(meth)acrylat, 1,10-Decan-dioldi(meth)acrylat, 1,12-Dodecandioldi(meth)acrylat, 2-Hydroxypropyl-1,3-di(meth)acrylat, 3-Hydroxypropyl-1,2-di(meth)acrylat, Neopentylglycoldi(meth)acrylat, 2,2-Bis[4-[3-(meth)acryloyloxy-2-hydroxypropoxy]phenyl]propan,

2,2-Bis[4-(meth)-acryloyloxyphenyl]propan, 2,2-Bis[4-(meth)acryloyloxyethoxyphenyl]propan, 2,2-Bis[4-(meth)acryloyloxydiethoxyphenyl]propan, 2,2-Bis[4-(meth)acryloyloxytriethoxy-phenyl]propan 2,2-Bis[4-(meth)acryloyloxytetraethoxyphenyl]propan, 2,2-Bis[4-(meth)acryloyloxypentaethoxyphenyl]-propan, 2,2-Bis[4-(meth)acryloyloxydipropoxy-phenyl]propan, 2,2-Bis[4-(meth)acryloyloxyethoxyphenyl]-2-[4-(meth)acryloyloxy-diethoxyphenyl]propan, 2-[4-(Meth)acryloyloxydiethoxyphenyl]-2-[4-(meth)-acryl-oyloxytriethoxyphenyl]propan, 2-[4-(Meth)acryloyloxydipropoxyphenyl]-2-[4-(meth)-acryloyloxytriethoxyphenyl]propan, 2,2-Bis[4-(meth)acryloyloxyisopropoxyphenyl]-propan, ethoxyliertes Bisphenol-A-di(meth)acrylat, propoxyliertes Bisphenol-A-di(meth)acrylat, 3(4),8(9)-Bis((meth)acryloyloxymethyl)tricyclo[5.2.1.0$^{2,6}$]decan.

16. Dentale, radikalisch polymerisierbare Zusammensetzung nach einem der vorangehenden Aspekte, umfassend (A4) in einer Menge von 1 bis 20 Gew.-%, bevorzugt 1 bis 10 Gew.-%, bezogen auf die Gesamtmasse der polymerisierbaren Zusammensetzung.

17. Dentale, radikalisch polymerisierbare Zusammensetzung nach einem der vorangehenden Aspekte, wobei die multifunktionelle (Meth)acryl-Verbindung (A4) ausgewählt ist aus der Gruppe bestehend aus Glyceroltri(meth)acrylat, ethoxyliertes, Glyceroltri(meth)acrylat, propoxyliertes, Glyceroltri(meth)acrylat, Trimethylol-ethantri(meth)acrylat, ethoxyliertes Trimethylolethantri(meth)acrylat, Trimethylol-propantri(meth)acrylat, ethoxyliertes Trimethylolpropantri(meth)acrylat, Ditri-methylolpropantri(meth)acrylat, Ditri methylo lpropa ntetra (meth)acrylat, Penta-erythritoltri(meth)acrylat, Pentaerythritoltetra(meth)acrylat, ethoxyliertes Penta-erythritoltetra(meth)acrylat, Dipentaerythritoltri(meth)acrylat, Dipentaerythritoltetra-(meth)acrylat, Dipentaerythritolpenta(meth)acrylat, Dipentaerythritolhexa(meth)-acrylat, ethoxyliertes Dipentaerythritolpenta(meth)acrylat, ethoxyliertes Dipenta-erythritolhexa(meth)acrylat und (2,4,6-Trioxo-1,3,5-triazin-1,3,5(2H,4H,6H)-triyl)tri-2,1-ethandiyltri(meth)acrylat.

18. Dentale, radikalisch polymerisierbare Zusammensetzung nach einem der vorangehenden Aspekte, umfassend einen Photoinitiator oder Photoinitiatorsystem (B) in einer Menge von 0.1 bis 5 Gew.-%, bevorzugt 0.5 bis 4 Gew.-%, jeweils bezogen auf die Gesamtmasse der polymerisierbaren Zusammensetzung.

19. Dentale, radikalisch polymerisierbare Zusammensetzung nach einem der vorangehenden Aspekte, wobei (B) ausgewählt ist aus der Gruppe bestehend aus alpha-Diketonen, Benzoinalkylethern, Thioxanthonen, Benzophenonen, Acetophenonen, Acylphosphinoxiden, und Acylgermanium-Verbindungen.

20. Dentale, radikalisch polymerisierbare Zusammensetzung nach einem der vorangehenden Aspekte, wobei (B) ausgewählt ist aus der Gruppe bestehend aus 1-Hydroxycyclohexylbenzophenon, 4-(2-Hydroxyethoxy)phenyl-(2-hydroxy-2-propyl)keton, 2-Hydroxy-2-methylpropiophenon, 2,4,6-Trimethylbenzoyldiphenylphosphinoxid, Phenyl-bis(2,4,6-trimethylbenzoyl)phosphinoxid, Ethyl phenyl(2,4,6-trimethylbenzoyl)phosphinate (CAS 84434-11-7) und (Poly(oxy-1,2-ethandiyl),α,α',α"-1,2,3-propantriyltris[ω-[[phenyl(2,4,6-trimethylbenzoyl)phosphinyl]oxy]-Polymer) (CAS 1834525-17-5), besonders bevorzugt ausgewählt aus der Gruppe bestehend aus 2,4,6-Trimethylbenzoyldiphenylphosphinoxid, Phenyl-bis(2,4,6-trimethylbenzoyl)phosphinoxid, Ethyl phenyl(2,4,6-trimethylbenzoyl)phosphinate und (Poly(oxy-1,2-ethandiyl),α,α',α"-1,2,3-propantriyltris[ω-[[phenyl(2,4,6-trimethylbenzoyl)phosphinyl]oxy]-Polymer).

21. Dentale, radikalisch polymerisierbare Zusammensetzung nach einem der vorangehenden Aspekte, umfassend einen oder mehrere Stabilisatoren (C) in einer Menge von 0.001 bis 1 Gew.-%, bevorzugt 0.01 bis 0.5 Gew.-%, besonders bevorzugt 0.01 bis 0.3 Gew.-%, jeweils bezogen auf die Gesamtmasse der polymerisierbaren Zusammensetzung.

22. Dentale, radikalisch polymerisierbare Zusammensetzung nach einem der vorangehenden Aspekte, wobei die Stabilisatoren (C) ausgewählt sind aus der Gruppe bestehend aus Hydrochinon, Hydrochinonmonomethylether, 2,6-Di-tert.-butyl-4-methylphenol, tert.-Butylhydroxyanisol und 2,2,6,6-Tetramethyl-piperidin-1-oxyl.

23. Dentale, radikalisch polymerisierbare Zusammensetzung nach einem der vorangehenden Aspekte, umfassend

A1) in einer Menge von 30 bis 70 Gew.-%, bevorzugt 40 bis 60 Gew.-%,
A2) in einer Menge von 10 bis 50 Gew.-%, bevorzugt 20 bis 50 Gew.-%,
A3) in einer Menge von 1 bis 30 Gew.-%, bevorzugt 2 bis 15 Gew.-% und
B) in einer Menge von 0.1 bis 5 Gew.-%, bevorzugt 0.5 bis 4 Gew.-%,

jeweils bezogen auf die Gesamtmasse der polymerisierbaren Zusammensetzung.

24. Dentale, radikalisch polymerisierbare Zusammensetzung nach einem der vorangehenden Aspekte, umfassend

A1) in einer Menge von 30 bis 70 Gew.-%, bevorzugt 40 bis 60 Gew.-%,
A2) in einer Menge von 10 bis 50 Gew.-%, bevorzugt 20 bis 50 Gew.-%,
A3) in einer Menge von 1 bis 30 Gew.-%, bevorzugt 2 bis 15 Gew.-%,
A4) in einer Menge von 1 bis 20 Gew.-%, bevorzugt 1 bis 10 Gew.-% und
B) in einer Menge von 0.1 bis 5 Gew.-%, bevorzugt 0.5 bis 4 Gew.-%,

jeweils bezogen auf die Gesamtmasse der polymerisierbaren Zusammensetzung.

25. Dentale, radikalisch polymerisierbare Zusammensetzung nach einem der vorangehenden Aspekte, umfassend

A1) in einer Menge von 30 bis 70 Gew.-%, bevorzugt 40 bis 60 Gew.-%,
A2) in einer Menge von 10 bis 50 Gew.-%, bevorzugt 20 bis 50 Gew.-%,
A3) in einer Menge von 1 bis 30 Gew.-%, bevorzugt 2 bis 15 Gew.-%,
A4) in einer Menge von 1 bis 20 Gew.-%, bevorzugt 1 bis 10 Gew.-%,
B) in einer Menge von 0.1 bis 5 Gew.-%, bevorzugt 0.5 bis 4 Gew.-% und
C) in einer Menge von 0.001 bis 1 Gew.-%, bevorzugt 0.01 bis 0.5 Gew.-%,

jeweils bezogen auf die Gesamtmasse der polymerisierbaren Zusammensetzung.

26. Dentale, radikalisch polymerisierbare Zusammensetzung nach einem der vorangehenden Aspekte, wobei

die (Meth)acryl-Verbindung (A1) mindestens eine Urethangruppe enthält und/oder (vorzugsweise und)
die (Meth)acryl-Verbindung (A2) keine Urethangruppe enthält und/oder (vorzugsweise und)
die (Meth)acryl-Verbindung (A3) keine Urethangruppe enthält und/oder (vorzugsweise und)
die (Meth)acryl-Verbindung (A4) keine Urethangruppe enthält.

27. Dentale, radikalisch polymerisierbare Zusammensetzung nach einem der vorangehenden Aspekte, zur Verwendung in Stereolithographie und/oder 3D-Druck, vorzugsweise zum 3D-Druck von dentalen Formteilen, bevorzugt zum 3D-Druck von dentalen Transferschienen, IBTs, Übertragungsschlüsseln und Bohrschablonen.

28. Dentale, radikalisch polymerisierbare Zusammensetzung nach einem der vorangehenden Aspekte, zur Anwendung in einem Verfahren zur therapeutischen oder chirurgischen Behandlung des menschlichen Körpers, vorzugsweise zur spezifischen Anwendung in einem therapeutischen Verfahren zur Korrektur von Zahnfehlstellungen oder in einem chirurgischen Verfahren zum Bohren von Implantatlöchern.

29. Verwendung einer dentalen, radikalisch polymerisierbaren Zusammensetzung, nach einem der vorangehenden Aspekte, in Stereolithographie und/oder 3D-Druck, vorzugsweise zum 3D-Druck von dentalen Formteilen, bevorzugt zum 3D-Druck von dentalen Transferschienen, IBTs, Übertragungsschlüsseln und Bohrschablonen.

## Patentansprüche

**1.** Dentale, radikalisch polymerisierbare Zusammensetzung umfassend

A1) eine oder mehrere polymerisierbare (Meth)acryl-Verbindungen der Formel I

$$Y\text{-}O\text{-}[(C_3H_6O)_v\text{-}L^1]_n\text{-}(C_3H_6O)_w\text{-}Z \qquad (I)$$

wobei

$Y = H_2C{=}C(R^1)\text{-}C({=}O)\text{-}$ oder $H_2C{=}C(R^1)\text{-}C({=}O)\text{-}X\text{-}L^2\text{-}$,
$R^1$ = H oder $CH_3$,
X = O oder NH,
$L^1$ = divalente organische Linkergruppe,
$L^2$ = divalente organische Linkergruppe,
Z = Y, H oder C1 bis C4-Alkyl,

v = 36 bis 100,
w = 36 bis 200,
n = 0 bis 3,
n*v+w = 36 bis 200

A2) eine oder mehrere monofunktionelle (Meth)acryl-Verbindungen mit einer (Meth)acryl-Gruppe, die nicht der Formel I entsprechen,
A3) eine oder mehrere difunktionelle (Meth)acryl-Verbindungen mit zwei (Meth)acryl-Gruppen, die nicht der Formel I entsprechen und
B) einen Photoinitiator oder ein Photoinitiatorsystem.

**2.** Dentale, radikalisch polymerisierbare Zusammensetzung nach Anspruch 1, zusätzlich umfassend
A4) eine oder mehrere multifunktionelle (Meth)acryl-Verbindungen mit drei bis sechs (Meth)acryl-Gruppen.

**3.** Dentale, radikalisch polymerisierbare Zusammensetzung nach einem der vorangehenden Ansprüche, umfassend

A1) in einer Menge von 30 bis 70 Gew.-%, bevorzugt 40 bis 60 Gew.-%,
A2) in einer Menge von 10 bis 50 Gew.-%, bevorzugt 20 bis 50 Gew.-%,
A3) in einer Menge von 1 bis 30 Gew.-%, bevorzugt 2 bis 15 Gew.-% und
B) in einer Menge von 0.1 bis 5 Gew.-%, bevorzugt 0.5 bis 4 Gew.-%,

jeweils bezogen auf die Gesamtmasse der polymerisierbaren Zusammensetzung.

**4.** Dentale, radikalisch polymerisierbare Zusammensetzung nach einem der vorangehenden Ansprüche, umfassend

A1) in einer Menge von 30 bis 70 Gew.-%, bevorzugt 40 bis 60 Gew.-%,
A2) in einer Menge von 10 bis 50 Gew.-%, bevorzugt 20 bis 50 Gew.-%,
A3) in einer Menge von 1 bis 30 Gew.-%, bevorzugt 2 bis 15 Gew.-%,
A4) in einer Menge von 1 bis 20 Gew.-%, bevorzugt 1 bis 10 Gew.-% und
B) in einer Menge von 0.1 bis 5 Gew.-%, bevorzugt 0.5 bis 4 Gew.-%,

jeweils bezogen auf die Gesamtmasse der polymerisierbaren Zusammensetzung.

**5.** Dentale, radikalisch polymerisierbare Zusammensetzung nach einem der vorangehenden Ansprüche, wobei in der (Meth)acryl-Verbindung der Formel I

X=O,
Z = Y,
n = 0 und
w = 36 bis 200, bevorzugt 75 bis 180, besonders bevorzugt 75 bis 150, ganz besonders bevorzugt 100 bis 150.

**6.** Dentale, radikalisch polymerisierbare Zusammensetzung nach einem der vorangehenden Ansprüche, wobei in der (Meth)acryl-Verbindung der Formel I

X=O,
Z = Y,
n = 1 bis 3,
v = 36 bis 100, bevorzugt 36 bis 90, besonders bevorzugt 36 bis 80,
w = 36 bis 100, bevorzugt 36 bis 90, besonders bevorzugt 36 bis 80 und
v+w = 75 bis 180, bevorzugt 75 bis 150, besonders bevorzugt 100 bis 150.

**7.** Dentale, radikalisch polymerisierbare Zusammensetzung nach einem der vorangehenden Ansprüche, wobei in der (Meth)acryl-Verbindung der Formel I $L^1$ eine geradkettige, verzweigte und/oder cyclische Alkylen-, Arylen-, Arylalkylen- oder Alkylarylengruppe ist, die -O-, -OC(=O)-, -C(=O)O-, -NHC(=O)-, -C(=O)NH-, -OC(=O)NH-, oder -NHC(=O)O- enthalten kann, und $L^2$ eine geradkettige, verzweigte und/oder cyclische Alkylen-, Arylen-, Arylalkylen- oder Alkylarylengruppe ist, die -O-, -OC(=O)-, -C(=O)O-, -NHC(=O)-, -C(=O)NH-, -OC(=O)NH-, oder -NHC(=O)O- enthalten kann, vorzugsweise ist $L^1$ und/oder $L^2$ ausgewählt aus der Gruppe bestehend aus

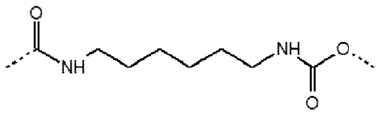, 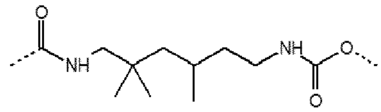,

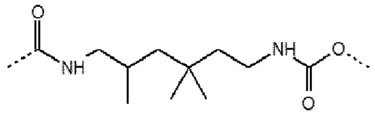, 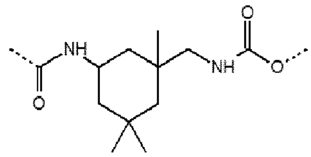,

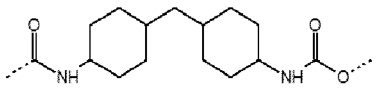, 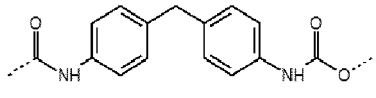,

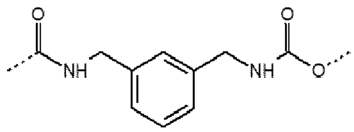 und 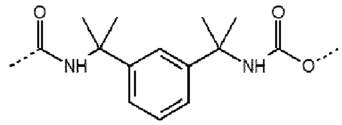,

bevorzugt ausgewählt aus der Gruppe bestehend aus

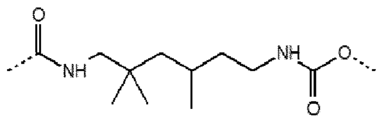, 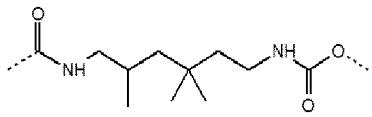

und

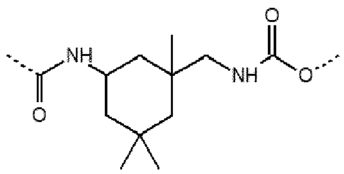,

wobei $L^1$ mit der linken gestrichelten Linie an das äußere Sauerstoffatom der Polypropylengruppe $(C_3H_6O)_v$ gebunden ist und mit der rechten gestrichelten Linie an das äußere Kohlenstoffatom der Polypropylengruppe $(C_3H_6O)_w$ gebunden ist und/oder
wobei $L^2$ mit der linken gestrichelten Linie an X gebunden ist und mit der rechten gestrichelten Linie über ein Sauerstoffatom ( -O- ) an das äußere Kohlenstoffatom einer der Polypropylengruppen $(C_3H_6O)_w$ oder $(C_3H_6O)_v$ gebunden ist.

**8.** Dentale, radikalisch polymerisierbare Zusammensetzung nach einem der vorangehenden Ansprüche, wobei die (Meth)acryl-Verbindung der Formel I ein Molekulargewicht

von größer 5000 g/mol, bevorzugt von größer 6000 g/mol, besonders bevorzugt von größer 7000 g/mol, und/oder
im Bereich von 5000 bis 15000 g/mol, bevorzugt im Bereich von 6000 bis 12000 g/mol, besonders bevorzugt im Bereich von 7000 bis 10000 g/mol,
aufweist.

**9.** Dentale, radikalisch polymerisierbare Zusammensetzung nach einem der vorangehenden Ansprüche, wobei die

monofunktionelle (Meth)acryl-Verbindung (A2) ausgewählt ist aus der Gruppe bestehend aus 2-Hydroxyethyl(meth)acrylat, 2-Hydroxypropyl(meth)acrylat, 3-Hydroxypropyl(meth)acrylat, Tetrahydrofurfuryl(meth)acrylat, 4-(Meth)acryloylmorpholin, Cyclohexyl(meth)acrylat, 3,3,5-Trimethylcyclohexyl(meth)acrylat, 4-(1,1-Dimethylethyl)cyclohexyl(meth)acrylat, Isobornyl(meth)acrylat, (Octahydro-4,7-methano-1H-indenyl)methyl(meth)acrylat, Benzyl(meth)acrylat, 2-Phenoxyethyl(meth)acrylat, 2-(2-Phenoxyethoxy)-ethyl(meth)acrylat, 2-[2-(2-Phenoxyethoxy)ethoxy]ethyl(meth)acrylat, ethoxyliertes 2-Phenoxyethyl(meth)acrylat 2-Ethoxyethyl(meth)acrylat, 2-(2-Ethoxyethoxy)-ethyl(meth)acrylat, 2-[2-(2-Ethoxyethoxy)ethoxy]ethyl(meth)acrylat, 2-Methoxyethyl(meth)acrylat, 2-(2-Methoxyethoxy)ethyl(meth)acrylat, 2-[2-(2-Methoxy-ethoxy)ethoxy]ethyl(meth)acrylat, Hexyl(meth)acrylat, 2-Ethylhexyl(meth)acrylat, Decyl(meth)acrylat, Isodecyl(meth)acrylat, Dodecyl(meth)acrylat, 2-Hydroxy-3-(prop-2-enoyloxy)propyl 2-methyl-2-propylhexanoat, und (5-Ethyl-1,3-dioxan-5-yl)methyl(meth)acrylat.

**10.** Dentale, radikalisch polymerisierbare Zusammensetzung nach einem der vorangehenden Ansprüche, wobei die difunktionelle (Meth)acryl-Verbindung (A3) ausgewählt ist aus der Gruppe bestehend aus Ethylenglycoldi(meth)acrylat, Diethylenglycoldi(meth)acrylat, Triethylenglycoldi(meth)acrylat, Tetraethylenglycol-di(meth)acrylat, Polyethylenglycoldi(meth)acrylat, Propylenglycoldi(meth)acrylat, Dipropylenglycoldi(meth)acrylat, Tripropylenglycoldi(meth)acrylat, Tetrapropylen-glycoldi(meth)acrylat, Polypropylenglycoldi(meth)acrylat, 1,4-Butandioldi-(meth)acrylat, 1,3-Butandioldi(meth)acrylat, 1,6-Hexandioldi(meth)acrylat, 1,10-Decandioldi(meth)acrylat, 1,12-Dodecandioldi(meth)acrylat, 2-Hydroxypropyl-1,3-di(meth)acrylat, 3-Hydroxypropyl-1,2-di(meth)acrylat, Neopentylglycoldi(meth)-acrylat, 2,2-Bis[4-[3-(meth)acryloyloxy-2-hydroxypropoxy]phenyl]propan, 2,2-Bis[4-(meth)acryloyloxyphenyl]propan, 2,2-Bis[4-(meth)acryloyloxyethoxyphenyl]propan, 2,2-Bis[4-(meth)acryloyloxydiethoxyphenyl]propan, 2,2-Bis[4-(meth)acryloyloxy-triethoxyphenyl]propan 2,2-Bis[4-(meth)acryloyloxytetraethoxyphenyl]propan, 2,2-Bis[4-(meth)acryloyloxypentaethoxyphenyl]-propan, 2,2-Bis[4-(meth)acryloyloxy-dipropoxyphenyl]propan, 2,2-Bis[4-(meth)acryloyloxyethoxyphenyl]-2-[4-(meth)-acryloyloxydiethoxyphenyl]propan, 2-[4-(Meth)acryloyloxydiethoxyphenyl]-2-[4-(meth)-acryloyloxytriethoxyphenyl]propan, 2-[4-(Meth)acryloyloxydipropoxyphenyl]-2-[4-(meth)acryloyloxytriethoxyphenyl]propan, 2,2-Bis[4-(meth)acryloyloxy-isopropoxyphenyl]propan, ethoxyliertes Bisphenol-A-di(meth)acrylat, propoxyliertes Bisphenol-A-di(meth)acrylat, 3(4),8(9)-Bis((meth)acryloyloxymethyl)tricyclo-[5.2.1.0$^{2,6}$]decan, 3,14-Dioxa-4,13-dioxo-5,12-diazahexadecan-1,16-diyldi-(meth)¬acrylat, 7,7,9-Trimethyl-3,14-dioxa-4,13-dioxo-5,12-diazahexadecan-1,16-diyldi¬(meth)acrylat, 7,9,9-Trimethyl-3,14-dioxa-4,13-dioxo-5,12-diazahexadecan-1,16-diyldi(meth)acrylat, 1,5,5-Trimethyl-1-[(2-(meth)acryloyloxyethyl)¬carbamoyl-methyl]-3-(2-(meth)acryloyloxyethyl)carbamoylcyclohexan (CAS 42405-01-6 Methacrylat; CAS 42404-50-2 Acrylat), 1,1'-[Methylenbis(4,1-phenylen¬imino-carbonyloxy-2,1-ethandiyl)]bis(meth)acrylat (CAS 51243-61-9 Methacrylat; CAS 69790-08-5 Acrylat), 1,1'-[Methylenbis(2,1-phenyleniminocarbonyloxy-2,1-ethandiyl)]bis(meth)acrylat.

**11.** Dentale, radikalisch polymerisierbare Zusammensetzung nach einem der vorangehenden Ansprüche, wobei die multifunktionelle (Meth)acryl-Verbindung (A4) ausgewählt ist aus der Gruppe bestehend aus Glyceroltri(meth)acrylat, ethoxyliertes, Glyceroltri(meth)acrylat, propoxyliertes, Glyceroltri(meth)acrylat, Trimethylolethantri(meth)acrylat, ethoxyliertes Trimethylolethantri(meth)acrylat, Trimethylolpropantri(meth)acrylat, ethoxyliertes Trimethylolpropantri(meth)acrylat, Ditrimethylolpropantri(meth)acrylat, Ditrimethylolpropantetra(meth)acrylat, Penta-erythritoltri(meth)acrylat, Pentaerythritoltetra(meth)acrylat, ethoxyliertes Penta-erythritoltetra(meth)acrylat, Dipentaerythritoltri(meth)acrylat, Dipentaerythritoltetra-(meth)acrylat, Dipentaerythritolpenta(meth)acrylat, Dipentaerythritolhexa(meth)-acrylat, ethoxyliertes Dipentaerythritolpenta(meth)acrylat, ethoxyliertes Dipentaerythritolhexa(meth)acrylat und (2,4,6-Trioxo-1,3,5-triazin-1,3,5(2H,4H,6H)-triyl)tri-2,1-ethandiyltri(meth)acrylat.

**12.** Dentale, radikalisch polymerisierbare Zusammensetzung nach einem der vorangehenden Ansprüche, wobei der Photoinitiator (B) ausgewählt ist aus der Gruppe bestehend aus 1-Hydroxycyclohexylbenzophenon, 4-(2-Hydroxyethoxy)phenyl-(2-hydroxy-2-propyl)keton, 2-Hydroxy-2-methylpropio-phenon, 2,4,6-Trimethylbenzoyldiphenylphosphinoxid, Phenyl-bis(2,4,6-tri-methybenzoyl)phosphinoxid, Ethyl phenyl(2,4,6-trimethylbenzoyl)phosphinate (CAS 84434-11-7) und (Poly(oxy-1,2-ethandiyl),$\alpha,\alpha',\alpha''$-1,2,3-propantriyltris[w-[[phenyl(2,4,6-trimethylbenzoyl)phosphinyl]oxy]-Polymer) (CAS 1834525-17-5), besonders bevorzugt ausgewählt aus der Gruppe bestehend aus 2,4,6-Trimethylbenzoyldiphenylphosphinoxid, Phenyl-bis(2,4,6-trimethylbenzoyl)-phosphinoxid, Ethyl phenyl(2,4,6-trimethylbenzoyl)phosphinate und (Poly(oxy-1,2-ethandiyl),$\alpha,\alpha',\alpha''$-1,2,3-propantriyltris[$\omega$-[[phenyl(2,4,6-trimethylbenzoyl)phosphin-yl]oxy]-Polymer).

**13.** Dentale, radikalisch polymerisierbare Zusammensetzung nach einem der vorangehenden Ansprüche, wobei

die (Meth)acryl-Verbindung (A1) mindestens eine Urethangruppe enthält
und/oder
die (Meth)acryl-Verbindung (A2) keine Urethangruppe enthält und/oder

die (Meth)acryl-Verbindung (A3) keine Urethangruppe enthält
und/oder
die (Meth)acryl-Verbindung (A4) keine Urethangruppe enthält.

**14.** Dentale, radikalisch polymerisierbare Zusammensetzung nach einem der vorangehenden Ansprüche, zur Verwendung in Stereolithographie und/oder 3D-Druck, vorzugsweise zum 3D-Druck von dentalen Formteilen, bevorzugt zum 3D-Druck von dentalen Transferschienen, IBTs, Übertragungsschlüsseln und Bohrschablonen.

**15.** Verwendung einer dentalen, radikalisch polymerisierbaren Zusammensetzung nach einem der vorangehenden Ansprüche in Stereolithographie und/oder 3D-Druck, vorzugsweise zum 3D-Druck von dentalen Formteilen, bevorzugt zum 3D-Druck von dentalen Transferschienen, IBTs, Übertragungsschlüsseln und Bohrschablonen.

Europäisches Patentamt
European Patent Office
Office européen des brevets

# EUROPÄISCHER RECHERCHENBERICHT

Nummer der Anmeldung
EP 23 19 5901

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X,D | WO 2019/175716 A1 (3M INNOVATIVE PROPERTIES CO [US]) 19. September 2019 (2019-09-19)<br>* Seite 1, Zeile 28 - Seite 2, Zeile 9 *<br>* Seite 2, Zeile 33 *<br>* Seite 59; Beispiel 3 *<br>* Seite 61; Beispiel 6 *<br>* Seite 66, Zeile 5 - Seite 67, Zeile 2; Beispiel 24 *<br>* Tabelle 1 *<br>* Anspruch 2 *<br>----- | 1-15 | INV.<br>A61C7/08<br>A61K6/62<br>A61K6/887<br>B33Y70/00<br>B33Y80/00 |
| X | CN 112 812 265 A (ANQING FEIKAI NEW MAT CO LTD) 18. Mai 2021 (2021-05-18) | 1,3,<br>5-10,<br>12-14 | |
| A | * Synthesebeispiel 7 *<br>* Beispiel 4 *<br>----- | 2,4,11,<br>15 | |

RECHERCHIERTE SACHGEBIETE (IPC)

A61C
A61K
B33Y

Der vorliegende Recherchenbericht wurde für alle Patentansprüche erstellt

2

| Recherchenort | Abschlußdatum der Recherche | Prüfer |
|---|---|---|
| Den Haag | 1. Februar 2024 | Grave, Christian |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
.....................................................................................................
& : Mitglied der gleichen Patentfamilie, übereinstimmendes Dokument

EPO FORM 1503 03.82 (P04C03)

## ANHANG ZUM EUROPÄISCHEN RECHERCHENBERICHT ÜBER DIE EUROPÄISCHE PATENTANMELDUNG NR.

EP 23 19 5901

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

01-02-2024

| Im Recherchenbericht angeführtes Patentdokument | Datum der Veröffentlichung | Mitglied(er) der Patentfamilie | Datum der Veröffentlichung |
|---|---|---|---|
| WO 2019175716 A1 | 19-09-2019 | CN 111868125 A | 30-10-2020 |
| | | EP 3765537 A1 | 20-01-2021 |
| | | JP 2021518449 A | 02-08-2021 |
| | | US 2020332046 A1 | 22-10-2020 |
| | | WO 2019175716 A1 | 19-09-2019 |
| CN 112812265 A | 18-05-2021 | KEINE | |

EPO FORM P0461

Für nähere Einzelheiten zu diesem Anhang : siehe Amtsblatt des Europäischen Patentamts, Nr.12/82

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 9729901 A1
- WO 2013153183 A2
- WO 2019175716 A1
- WO 2022097667 A1


### In der Beschreibung aufgeführte Nicht-Patentliteratur


- *CHEMICAL ABSTRACTS,* 42405-01-6
- *CHEMICAL ABSTRACTS,* 42404-50-2
- *CHEMICAL ABSTRACTS,* 51243-61-9
- *CHEMICAL ABSTRACTS,* 69790-08-5
- *CHEMICAL ABSTRACTS,* 84434-11-7
- *CHEMICAL ABSTRACTS,* 1834525-17-5
- *CHEMICAL ABSTRACTS,* 822-06-0
- *CHEMICAL ABSTRACTS,* 32052-51-0
- *CHEMICAL ABSTRACTS,* 4098-71-9
- *CHEMICAL ABSTRACTS,* 77-58-7
- *CHEMICAL ABSTRACTS,* 128-37-0
- *CHEMICAL ABSTRACTS,* 7534-94-3
- *CHEMICAL ABSTRACTS,* 5117-12-4
- *CHEMICAL ABSTRACTS,* 2455-24-5
- *CHEMICAL ABSTRACTS,* 10595-06-9
- *CHEMICAL ABSTRACTS,* 868-77-9
- *CHEMICAL ABSTRACTS,* 66492-51-1
- *CHEMICAL ABSTRACTS,* 41637-38-1
- *CHEMICAL ABSTRACTS,* 43048-08-4
- *CHEMICAL ABSTRACTS,* 6606-59-3
- *CHEMICAL ABSTRACTS,* 72869-86-4
- *CHEMICAL ABSTRACTS,* 4986-89-4
- *CHEMICAL ABSTRACTS,* 51728-26-8
- *CHEMICAL ABSTRACTS,* 15625-89-5
- *CHEMICAL ABSTRACTS,* 28961-43-5
- *CHEMICAL ABSTRACTS,* 40220-08-4
- *CHEMICAL ABSTRACTS,* 75980-60-8